# EUROPEAN PATENT APPLICATION

(11) **EP 2 787 350 A1**
(43) Date of publication of application: **08.10.2014**
(21) Application number: 13162509.7
(22) Date of filing: 05.04.2013
(51) Int. Cl.: G01N 33/574

(54) **ASRGL1 in endometrial cancer**

(71) Applicant: Atlas Antibodies AB, 106 91 Stockholm (SE)
(72) Inventor: Edqvist, Per-Henrik, SE-757 58 Uppsala (SE); Pontén, Fredrik, SE-752 37 Uppsala (SE)
(74) Representative: Kransell & Wennborg KB

(57) **Abstract**

There is provided a method for determining whether a mammalian subject having an endometrial cancer belongs to a first or a second group, wherein the prognosis of subjects of the first group is better than the prognosis of subjects of the second group, comprising the steps of:
a) evaluating an amount of ASRGL1 in at least part of a sample earlier obtained from the subject and determining a sample value corresponding to the evaluated amount;
b) comparing said sample value with a predetermined reference value; and
if said sample value is higher than said reference value,
c1) concluding that the subject belongs to the first group; and
if said sample value is lower than or equal to said reference value, c2) concluding that the subject belongs to the second group.

## Description

### Technical field

The present disclosure relates to the field of endometrial cancer prognosis.

### Background

### Cancer

Cancer is one of the most common diseases, and a major cause of death, in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of biopsy material from suspected tumors remains the golden standard for cancer diagnostics. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is crucial for a correct diagnosis and may provide an instrument to predict a prognosis. The diagnostic and prognostic information for a specific tumor subsequently determines an adequate therapeutic strategy for a given cancer patient. A commonly used method, in addition to histochemical staining of tissue sections, to obtain more information regarding a tumor is immunohistochemical staining. IHC allows for the detection of protein expression patterns in tissues and cells using specific antibodies. The use of IHC in clinical diagnostics allows for the detection of immunoreactivity in different cell populations, in addition to the information regarding tissue architecture and cellular morphology that is assessed from the histochemically stained tumor tissue section. IHC can be involved in supporting the accurate diagnosis, including staging and grading, of a primary tumor as well as in the diagnostics of metastases of unknown origin. The most commonly used antibodies in clinical practice today include antibodies against cell type "specific" proteins, e.g., PSA (prostate), MelanA (melanocytes) and Thyroglobulin (thyroid gland), and antibodies recognizing intermediate filaments (epithelial, mesenchymal, glial), cluster of differentiation (CD) antigens (hematopoetic, sub-classification of lympoid cells) and markers of malignant potential, e.g., Ki67 (proliferation), p53 (commonly mutated tumor suppressor gene) and HER-2 (growth factor receptor).

Aside from IHC, the use of *in situ* hybridization for detecting gene amplification and gene sequencing for mutation analysis are evolving technologies within cancer diagnostics. In addition, global analysis of transcripts, proteins or metabolites adds relevant information. However, most of these analyses still represent basic research and have yet to be evaluated and standardized for the use in clinical medicine.

### Endometrial cancer

Endometrial cancer is currently the most common type of gynecological malignancy in Europe and North America, accounting for approximately 50 % of all gynecological cancers in these regions. Today, it is the fourth most common type of cancer for women in the regions mentioned above, but the incidence varies around the world, and in less developed countries the incidence of endometrial cancer may be ten times lower than in the developed world. In Sweden, about 1400 women are diagnosed with endometrial cancer each year, and the estimated incidence in Europe is almost 100,000 new cases in 2012. In the USA, it is estimated that almost 50,000 new endometrial cancer cases will be diagnosed during 2013, and that about 8,000 deaths will occur as a result of the disease.

Risk factors that have been identified for endometrial cancer include: Obesity, nulliparity, late menopause, diabetes, hypertension, and hereditary nonpolyposis colorectal cancer (HNPCC). Factors that decrease the risk of developing the disease include: Use of oral contraceptives, multiparity, smoking, and physical activity.

The most common histological type of endometrial carcinoma is the endometrioid adenocarcinoma, and approximately 80 % of all endometrial cancers belong to this type. Non-endometrioid subtypes include serous adenocarcinoma, clear-cell adenocarcinoma, and carcinosarcomas. Endometrial carcinoma is further clinically classified as belonging to either type I or type II. Most cases (about 80 %) are type I, associated with hyperestrongenism and good prognosis. Type II tumors are associated with non-hormone dependency, hormone receptor loss, and poor prognosis. These include poorly differentiated endometrioid carcinomas and the non-endometrioid subtypes.

The survival rate is generally high for endometrial cancer, with a relative 5-year survival rate of 84 %. However, 15-20 % of endometrial carcinomas recur, and the effect of systemic therapy is limited for metastatic disease.

### Diagnosis of endometrial cancer

Abnormal uterine bleeding occurs in more than 90 % of endometrial cancer patients, and contributes to early diagnosis of the disease with 75 % of diagnosed tumors still confined to the uterus. When endometrial cancer is suspected, a transvaginal ultrasound is usually performed in order to determine the endometrium thickness. In postmenopausal women, endometrium thickness of 4 mm or less indicates that presence of tumor is unlikely. If the endometrium is thicker, or immeasurable, tumor may be present, and an endometrial biopsy is normally performed. The biopsy material is analyzed, and if endometrial cancer is confirmed, further diagnostic imaging may be performed to assess myometrial infiltration and potential presence of metastatic spread.

Endometrioid carcinomas are graded according to the FIGO-system (International Federation of Gynecology and Obstetrics). Grade 1: Well formed glands with less than 5 % solid tumor areas; Grade 2: Carcinomas with 6-50 % solid tumor areas; Grade 3: Carcinomas with more than 50 % solid tumor areas. Serous- and clear-cell carcinomas are not graded, but defined as high grade.

Patients may be preoperatively stratified into high risk and low risk groups defined as follows: High risk: Non-endometrioid adenocarcinoma, or endometrioid adenocarcinoma grade 3, or more than 50 % myometrial invasion. Low risk: Endometrioid adenocarcinoma grade 1 or 2 or less than 50 % myometrial invasion. An intermediate risk group may be defined as follows: Endometrioid adenocarcinoma grade 1 or 2 with more than 50 % myometrial infiltration or endometrioid adenocarcinoma grade 3 with less than 50 % myometrial infiltration.

After surgery, the endometrial cancer is staged according to the FIGO-system. In stage I (T1, N0, M0), the tumor is confined to the corpus uteri, and in stage IA there is no myometrial invasion or less than 50 % myometrial infiltration, in stage IB there is more than 50 % myometrial infiltration. In stage II (T2, N0, M0), the tumor has invaded the cervical stroma, but has not spread beyond the uterus. In stage III (IIIA-IIIC2, T1-3, N0-2, M0), there is local or regional spread of the tumor. In stage IVA (T4, any N, M0), the tumor has spread to bladder or rectum, and in stage IVB (any T, any N, M1) there are distant metastases present.

Postoperatively, there is a new stratification of patients into risk groups as follows: Low risk: Endometrioid adenocarcinomas grade 1 and 2 with less than 50 % myometrial infiltration; Intermediate risk: Endometrioid adenocarcinoma grade 1 or 2 with more than 50 % myometrial infiltration or endometrioid adenocarcinoma grade 3 with less than 50 % myometrial infiltration; High risk: Non-endometrioid adenocarcinomas or endometrioid adenocarcinomas grade 3 with more than 50 % myometrial infiltration.

### Tumor markers

Few tumor markers exist for endometrial cancer today. Hormone receptor expression has been shown to be associated with good prognosis and may currently be used as a treatment predictive marker for hormonal treatment, but response rates vary greatly. Overexpression of some oncogenes, such as the HER2 receptor, stathmin, P53, and P16 has been associated with poor prognosis or aggressive phenotype, but none of these markers are routinely used in the clinic today.

### Prognostic factors

The most important prognostic factor is currently FIGO stage. Today, the 5-year survival for patients with stage I disease is approximately between 80 and 96 %, for stage II the survival is between 74 and 80 %, for stage III it is approximately between 50 and 60 %, and for stage IV the 5-year survival is as low as 20 %. The differentiation grade of the tumor and degree of myometrial infiltration are also important prognostic factors. Further, DNA content of the tumor cells has also been shown to be of prognostic importance. As mentioned above, stage I patients can be stratified into three prognostic groups, low-risk, intermediate-risk, and high-risk.

In WO 2005/005663, overexpression of the gene CRASH (also referred to as ASRGL1) is associated with a potential for tumor progression, in particular in a gynecological cancer or a prostate cancer. The teachings of the document are based on experimental results showing increased CRASH mRNA expression in uterine, ovarian, mammary and prostatic carcinoma compared to the corresponding normal tissues. Further, CRASH mRNA expression was detected in metastasizing, but not in non-metastasizing, colon cancer cell lines. High levels of ASRGL1 expression is thus correlated with an unfavorable prognosis.

### Treatment of endometrial cancer

Endometrial cancer is treated by primary surgery, where the uterus is removed by hysterectomy in combination with bilateral salpingo-oophorectomy (removal of the fallopian tubes and ovaries). For high risk patients (see above) it is also common to perform pelvic and periaortic lymphadenectomy. Patients who are not eligible for surgery are usually treated with radiation therapy.

After postoperative staging of the tumor and re-evaluation of patient risk group for stage I patients, postoperative adjuvant therapy may be considered. For low risk patients, no adjuvant treatment is deemed necessary. For intermediate risk patients, radiation therapy by vaginal brachytherapy may be a therapeutic option. However, while radiation therapy reduces the incidence of local and regional recurrence, improved survival has not been proven and the treatment is associated with considerable side-effects. High risk patients may be treated with chemotherapy in combination with radiation therapy, where external pelvic radiation therapy could be applied when no lymphadenectomy has been performed. Stage II patients may be treated with chemotherapy in combination with radiation therapy, where brachytherapy may be combined with external pelvic, or periaortic, radiation therapy in cases where no lymphadenectomy has been performed. Stage III patients may be treated with chemotherapy in combination with radiation therapy. For stage IV patients there is no standard chemotherapy option, but doxorubicin and paclitaxel are examples of chemotherapeutic substances that have shown antitumor activity. Hormonal treatment may be an option for stage IV patients, and progestational agents, such as hydroxyprogesterone, medroxyprogesterone, and megestrol, are most commonly used and have shown efficacy. Selective estrogen receptor modulators such as tamoxifen may also be used. For bulky pelvic disease, the treatment of choice is usually radiation therapy.

For recurrent disease, progestin therapy may be used for estrogen and progesterone receptor positive tumors. The use of tamoxifen is an option for patients who do not respond to progestin therapy.

### Summary

There is an object of the present invention to provide improvements related to endometrial cancer prognosis and treatment.

The following is a non-limiting and itemized listing of embodiments of the present disclosure, presented for the purpose of providing various features and combinations provided by the invention in certain of its aspects.
1. Method for determining whether a mammalian subject having an endometrial cancer belongs to a first or a second group, wherein the prognosis of subjects of the first group is better than the prognosis of subjects of the second group, comprising the steps of:
   a) evaluating an amount of ASRGL1 in at least part of a sample earlier obtained from the subject and determining a sample value corresponding to the evaluated amount;
   b) comparing said sample value with a predetermined reference value;
      and
      if said sample value is higher than said reference value,
      c1) concluding that the subject belongs to the first group; and
      if said sample value is lower than or equal to said reference value, c2) concluding that the subject belongs to the second group.
2. Method for determining a prognosis for a mammalian subject having an endometrial cancer, comprising the steps of:
   a) evaluating an amount of ASRGL1 in at least part of a sample earlier obtained from the subject and determining a sample value corresponding to the evaluated amount;
   b) comparing said sample value with a reference value associated with a reference prognosis; and
      if said sample value is higher than said reference value, c1)concluding that the prognosis for said subject is better than said
      reference prognosis; or
      if said sample value is lower than or equal to said reference value, c2)concluding that the prognosis for said subject is worse than or
      equal to said reference prognosis.
3. Method according to item 1 or 2, wherein the prognosis is a grade-independent prognosis.
4. Method for determining whether a subject having an endometrial cancer is not in need of an endometrial cancer treatment regimen, comprising the steps of:
   a) evaluating an amount of ASRGL1 in at least part of a sample earlier obtained from the subject and determining a sample value corresponding to the evaluated amount;
   b) comparing said sample value with a predetermined reference value;
      and
      if said sample value is higher than said reference value,
   c) concluding that said subject is not in need of the endometrial cancer treatment regimen.
5. Non-treatment strategy method for a subject having an endometrial cancer, comprising the steps of:
   a) evaluating an amount of ASRGL1 in at least part of a sample earlier obtained from the subject and determining a sample value corresponding to the evaluated amount;
   b) comparing said sample value with a predetermined reference value;
      and
      if said sample value is higher than said reference value,
   c) refraining from treating said subject with an endometrial cancer treatment regimen.
6. Method of treatment of a subject having an endometrial cancer, comprising the steps of:
   a) evaluating an amount of ASRGL1 in at least part of a sample earlier obtained from the subject and determining a sample value corresponding to the evaluated amount;
   b) comparing said sample value with a predetermined reference value;
      and
      if said sample value is lower than or equal to said reference value, c) treating said subject with an endometrial cancer treatment regimen.
7. Method according to item 6, further comprising the step of:
   d) refraining from treating said subject with the endometrial cancer treatment regimen if said sample value is higher than said reference value.
8. Method according to item 6, further comprising the step of:
   d') treating said subject with another endometrial cancer treatment regimen, which is less comprehensive than the treatment regimen of step c), if said sample value is higher than said reference value.
9. Method according to any one of items 5-8, wherein the endometrial cancer treatment regimen comprises hysterectomy in combination with lymphadenectomy.
10. Method according to any one of items 5-9, wherein the endometrial cancer treatment regimen comprises an adjuvant treatment.
11. Method according to item 10, wherein the adjuvant treatment comprises a chemotherapy.
12. Method according to item 9 or 10, wherein the adjuvant treatment comprises a radiation therapy, such as vaginal brachytherapy.
13. Method for determining whether a first or a second number of lymph nodes shall be removed from a mammalian subject having an endometrial cancer, wherein the first number is higher than the second number, comprising the steps of:
   a) evaluating an amount of ASRGL1 in at least part of a sample earlier obtained from the subject and determining a sample value corresponding to the evaluated amount;
   b) comparing said sample value with a predetermined reference value;
      and
      if said sample value is higher than said reference value,
      c1) concluding that the second number of lymph nodes shall be
      removed; and
      if said sample value is lower than or equal to said reference value, c2) concluding that the first number of lymph nodes shall be removed.
14. Method according to any one of items 1-13, wherein the endometrial cancer is an endometrioid adenocarcinoma.
15. Method according to any one of items 1-14, wherein the endometrial cancer is grade 1, 2 or 3.
16. Method according to item 15, wherein the endometrial cancer is grade 1 or 2 with more than 50 % myometrial infiltration or grade 3 with less than 50 % myometrial infiltration.
17. Method according to any one of items 1-16, wherein the endometrial cancer is stage I or II.
18. Method according to any one of the preceding items, wherein the sample comprises endometrial cancer tumor cells from said subject.
19. Method according to any one of the preceding items, wherein the sample is an endometrial tumor tissue sample.
20. Method according to any one of the preceding items, wherein the evaluation of step a) is limited to the cytoplasms of tumor cells of the sample.
21. Method according to any one of the preceding items, wherein said subject is a human.
22. Method according to any one of the preceding items, wherein said reference value is a value corresponding to a predetermined amount of ASRGL1 protein or ASRGL1 mRNA in a reference sample.
23. Method according to any preceding item, wherein the sample value of step a) is determined as being either 1, corresponding to detectable cytoplasmic ASRGL1 protein expression in tumor cells of the sample, or 0, corresponding to no detectable cytoplasmic ASRGL1 protein in tumor cells of the sample.
24. Method according to any preceding item, wherein the reference value of step b) corresponds to a reference sample having no detectable cytoplasmic ASRGL1 protein in tumor cells.
25. Method according to any preceding item, wherein the reference value of step b) is 0.
26. Method according to any preceding item, wherein the reference value is a cytoplasmic intensity, a cytoplasmic fraction or a function thereof.
27. Method according to any one of the preceding items, wherein the amino acid sequence of the ASRGL1 protein comprises a sequence selected from:
   i) SEQ ID NO:1; and
   ii) a sequence which is at least 85 % identical to SEQ ID NO:1.
28. Method according to any one of the preceding items, wherein the amino acid sequence of the ASRGL1 protein comprises or consists of a sequence selected from:
   i) SEQ ID NO:2 or 3; and
   ii) a sequence which is at least 85 % identical to SEQ ID NO:2 or 3.
29. Method according to any one of the preceding items, wherein step a) comprises:
   aI) applying to said sample of step a) a quantifiable affinity ligand capable of selective interaction with the ASRGL1 protein to be evaluated, said application being performed under conditions that enable binding of the affinity ligand to ASRGL1 protein present in the sample; and
   aII) quantifying the affinity ligand bound to said sample to evaluate said amount.
30. Method according to any one of items 1-28, wherein step a) comprises:
   a1) applying to said sample or step a) a quantifiable affinity ligand capable of selective interaction with the ASRGL1 protein to be quantified, said application being performed under conditions that enable binding of the affinity ligand to ASRGL1 protein present in the sample;
   a2) removing non-bound affinity ligand; and
   a3) quantifying affinity ligand remaining in association with the sample to evaluate said amount.
31. Method according to item 29 or 30, wherein the quantifiable affinity ligand is selected from the group consisting of antibodies, fragments thereof and derivatives thereof.
32. Method according to item 31, wherein the antibody fragments and derivatives are selected from the group consisting of Fab fragments, Fv fragments and single chain Fv (scFv) fragments.
33. Method according to item 31 or 32, wherein said quantifiable affinity ligand is obtainable by a process comprising a step of immunizing an animal with a peptide whose amino acid sequence consists of the sequence SEQ ID NO:1.
34. Method according to item 29 or 30, wherein said quantifiable affinity ligand is an oligonucleotide molecule.
35. Method according to item 29 or 30, wherein the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
36. Method according to any one of items 29-35, wherein said quantifiable affinity ligand is capable of selective interaction with a peptide whose amino acid sequence consists of the sequence SEQ ID NO:1.
37. Method according to any one of items 29-36, wherein the quantifiable affinity ligand comprises a label selected from the group consisting of fluorescent dyes and metals, chromophoric dyes, chemiluminescent compounds and bioluminescent proteins, enzymes, radioisotopes, particles and quantum dots.
38. Method according to any one of items 29-37, in which said quantifiable affinity ligand is detected using a secondary affinity ligand capable of recognizing the quantifiable affinity ligand.
39. Method according to item 38, in which said secondary affinity ligand capable of recognizing the quantifiable affinity ligand comprises a label selected from the group consisting of fluorescent dyes and metals, chromophoric dyes, chemiluminescent compounds and bioluminescent proteins, enzymes, radioisotopes, particles and quantum dots.
40. Use *ex vivo* of an ASRGL1 protein or an ASRGL1 mRNA molecule for identifying a favorable endometrial cancer prognosis.
41. Use according to item 40, wherein the favorable endometrial cancer prognosis is an expected five-year survival of at least 80 %.
42. Use according to item 41, wherein the favorable endometrial cancer prognosis is an expected five-year survival of at least 85 %, such as at least 90 %.
43. Use according to any one of items 40-42, wherein the ASRGL1 protein or ASRGL1 mRNA molecule is provided in a tumor tissue sample from the uterine glands.
44. Use according to any one of items 40-43, wherein the ASRGL1 protein consists of amino acid sequence SEQ ID NO:2 or 3 or an amino acid sequence which is at least 85 %, such as at least 90 %, such as at least 95 %, identical to SEQ ID NO:2 or 3.
45. Use of an affinity ligand capable of selective interaction with an ASRGL1 protein for identifying a favorable endometrial cancer prognosis.
46. Use according to item 45, wherein the favorable endometrial cancer prognosis is an expected five-year survival of at least 80 %.
47. Use according to item 46, wherein the favorable endometrial cancer prognosis is an expected five-year survival of at least 85 %, such as at least 90 %.
48. Use according to any one of items 45-47, wherein the affinity ligand is capable of selective interaction with a peptide whose amino sequence consist of SEQ ID NO:1.
49. Use according to any one of items 45-48, wherein the affinity ligand is selected from the group consisting of antibodies, fragments thereof and derivatives thereof.
50. Use according to item 49, wherein the antibody fragments and derivatives are selected from the group consisting of Fab fragments, Fv fragments and single chain Fv (scFv) fragments.
51. Use according to item 49, wherein the antibodies are monoclonal or polyclonal antibodies.
52. Use according to any one of items 45-51, wherein the ASRGL1 protein consists of amino acid sequence SEQ ID NO:2 or 3 or an amino acid sequence which is at least 85 %, such as at least 90 %, such as at least 95 %, identical to SEQ ID NO:2 or 3.

### Brief description of the figures

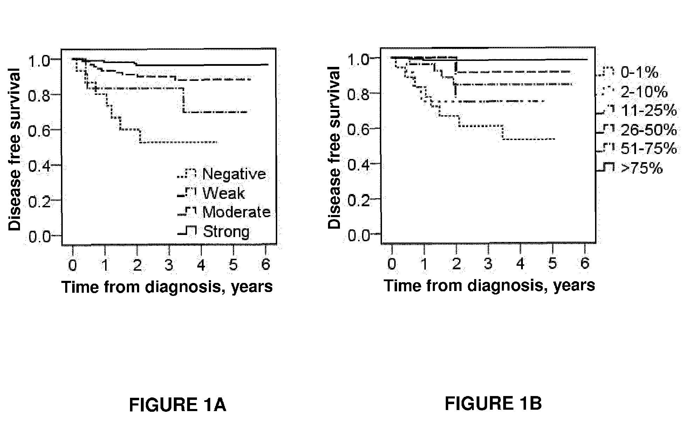

Figure 1 shows the impact of ASRGL1 protein level on disease free survival (DFS) of 220 patients diagnosed with endometrial cancer of the endometrioid type. Figure 1A shows DFS of patients stratified according to cytoplasmic intensity (CI), and Figure 1B shows DFS of patients stratified according to cytoplasmic fraction (CF).

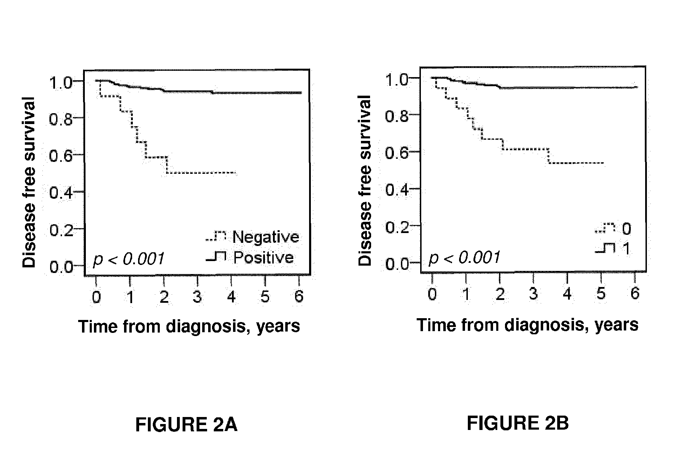

Figure 2 shows the impact of ASRGL1 protein level on DFS of the 220 endometrial cancer patients according to dichotomized variables for CI (Figure 2A) with a cut-off of negative (n=13) vs. positive (n=207) and CF (Figure 2B) where 0 represents a CF of < 2 % (n=19), and 1 represents a CF of ≥ 2 % (n=201).

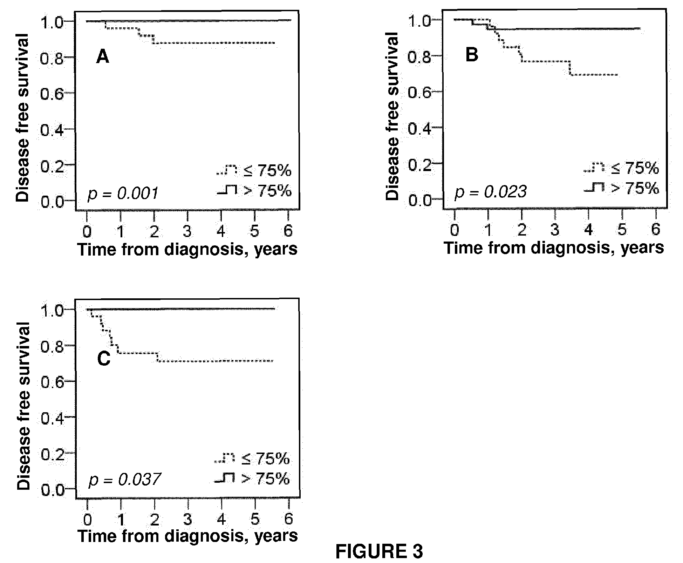

Figure 3 shows the grade-independent impact of ASRGL1 protein level on DFS of the 220 endometrial cancer patients according to dichotomized variables for CF (cut-off 75 %). Figure 3A shows DFS of patients with grade 1 tumors (for CF ≤ 75 % n=26, for CF > 75 % n=92), Figure 3B shows DFS of patients with grade 2 tumors (for CF ≤ 75 % n=26, for CF > 75 % n=37), and Figure 3C shows DFS of patients with grade 3 tumors (for CF ≤ 75 % n=26, for CF > 75 % n=13).

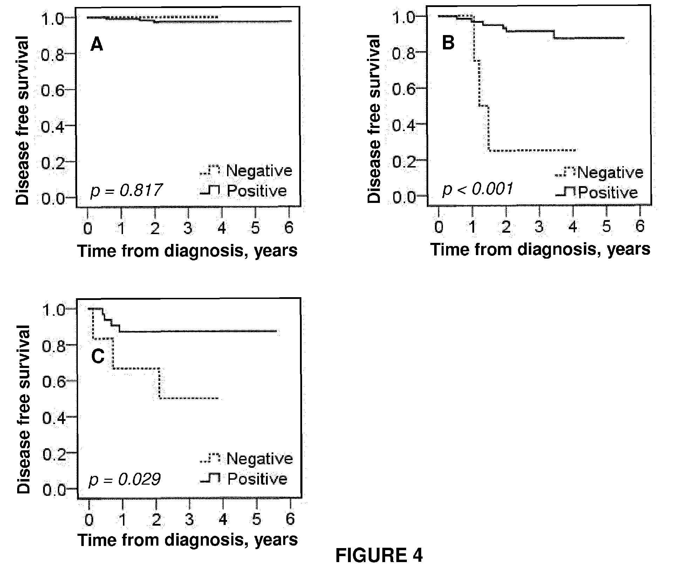

Figure 4 shows the grade-independent impact of ASRGL1 protein level on DFS of the 220 endometrial cancer patients according to dichotomized variables for CI (cut-off, negative vs. positive). Figure 4A shows DFS of patients with grade 1 tumors (for negative CI n=2, positive CI n=116), Figure 4B shows DFS of patients with grade 2 tumors (for negative CI n=4, positive CI n=59), and Figure 4C shows DFS of patients with grade 3 tumors (for negative CI n=7, positive CI n=32).

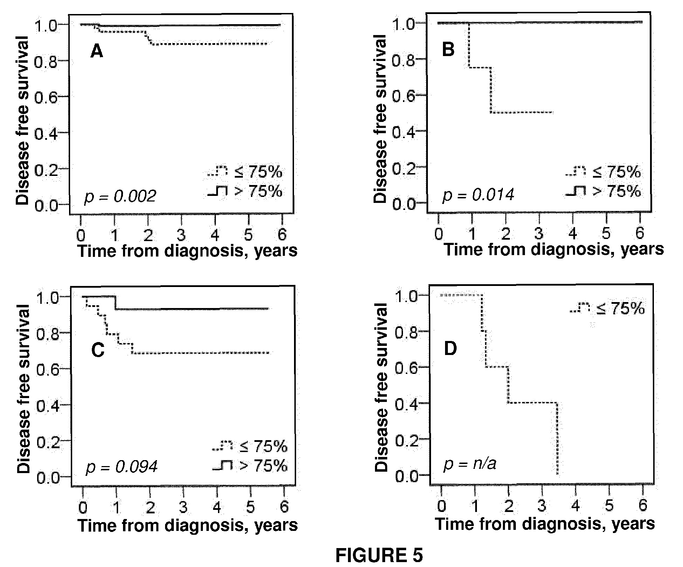

Figure 5 shows the stage-independent impact of ASRGL1 protein level on DFS of the 220 endometrial cancer patients according to dichotomized variables for CF (cut-off 75 %). Figure 5A shows DFS of patients with stage I disease (for CF ≤ 75 % n=49, for CF > 75 % n=117), Figure 5B shows DFS of patients with stage II disease (for CF ≤ 75 % n=4, for CF > 75 % n=11), Figure 5C shows DFS of patients with stage III disease (for CF ≤ 75 % n=20, for CF > 75 % n=14) and Figure 5D shows DFS of patients with stage IV disease (for CF ≤ 75 % n=5, for CF > 75 % n=0).

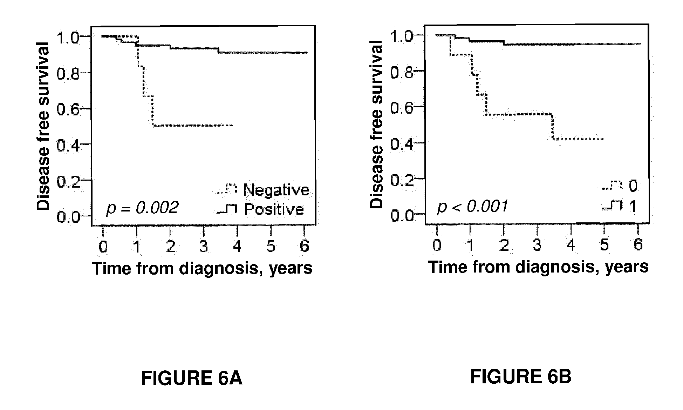

Figure 6 shows the impact of ASRGL1 protein level on DFS of 66 endometrial cancer patients belonging to an intermediate risk group as determined by having grade 1 or 2 tumors with > 50 % myometrial infiltration or grade 3 tumors with < 50 % myometrial infiltration. Patients are stratified according to dichotomized variables for CI (Figure 6A) with a cut-off of negative (n=6) vs. positive (n=60) and CF (Figure 6B) where 0 represents a CF of < 2 % (n=9), and 1 represents a CF of ≥ 2 % (n=57).

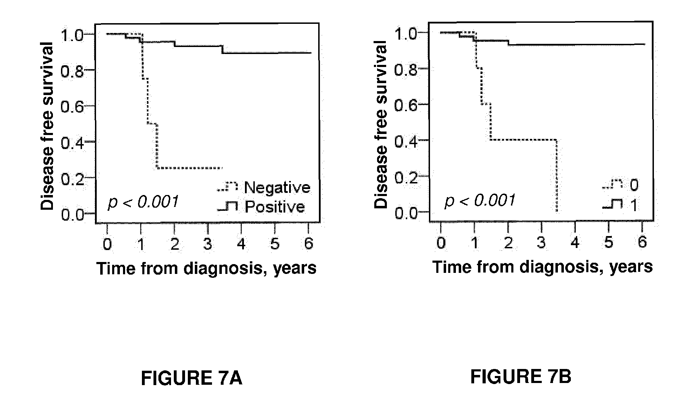

Figure 7 shows the impact of ASRGL1 protein level on DFS of 48 endometrial cancer patients belonging to the intermediate risk group that have grade 1 or 2 tumors with > 50 % myometrial infiltration. Patients are stratified according to dichotomized variables for CI (Figure 7A) with a cut-off of negative (n=4) vs. positive (n=44) and CF (Figure 7B) where 0 represents a CF of < 2 % (n=5), and 1 represents a CF of ≥ 2 % (n=43).

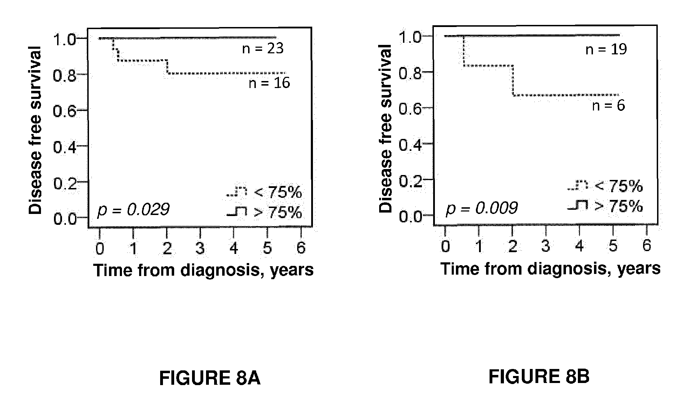

Figure 8 shows the impact of ASRGL1 protein level on DFS of stage I endometrial cancer patients belonging to an intermediate risk group. Figure 8A shows 39 stage I patients of an intermediate risk group defined as having grade 1 or 2 tumors with > 50 % myometrial infiltration or grade 3 tumors with < 50 % myometrial infiltration. Figure 8B shows 25 stage I patients of an intermediate risk group defined as having grade 1 or 2 tumors with > 50 % myometrial infiltration. Patients are stratified according to dichotomized variables for CF with a cut-off of 75 %.

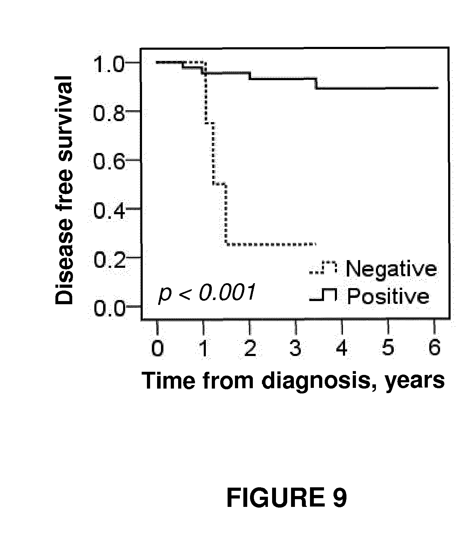

Figure 9 shows the impact of ASRGL1 protein level on DFS of 48 endometrial cancer patients belonging to the intermediate risk group that have grade 1 or 2 tumors with > 50 % myometrial infiltration. Patients are stratified according to dichotomized variables for the computed variable ClxCF, with a cut-off of negative (n = 4) vs. positive (n = 44).

### Detailed description

As a first aspect of the present disclosure, there is thus provided a method for determining whether a mammalian subject having an endometrial cancer belongs to a first or a second group, wherein the prognosis of subjects of the first group is better than the prognosis of subjects of the second group, comprising the steps of:
a) evaluating an amount of ASRGL1 in at least part of a sample earlier obtained from the subject and determining a sample value corresponding to the evaluated amount;
b) comparing said sample value with a predetermined reference value;
   and
   if said sample value is higher than said reference value,
c1)concluding that the subject belongs to the first group; and
   if said sample value is lower than or equal to said reference value,
c2)concluding that the subject belongs to the second group.

The present invention, based on an ASRGL1 level as an endometrial cancer status indicator, has a number of benefits. As well known by the person skilled in the art, a prognosis may be important for various reasons. The prognosis for an endometrial cancer subject generally reflects the aggressiveness of the cancer. Often, identification of the level of aggressiveness of an endometrial cancer is of vital importance as it helps a physician selecting an appropriate treatment strategy. The level of ASRGL1 expression may for example be used for identifying particularly aggressive forms of the cancer. In such cases, a more comprehensive treatment than what is normally considered may be applied. Thus, the subject may be given a painful or in any other sense unpleasant treatment, which normally is avoided, when the ASRGL1 level indicates that the cancer is aggressive. Also, the level of ASRGL1 expression may indicate a relatively favorable prognosis, which relieves the subject from a particular treatment. In other words, overtreatment may be avoided.

Today, it may for example be difficult to decide the extent of the surgery performed on a subject diagnosed with endometrial cancer. In particular, it may be difficult to decide whether or not to perform pelvic and periaortic lymphadenectomy. Also, the question may be if a higher or lower number of lymph nodes shall be removed. A method of the present disclosure may lead the physician to an informed decision in such a situation.

After surgery, it has to be decided whether or not to apply an adjuvant treatment. Again, a method according to the present disclosure may provide valuable guidance in such a decision, in particular for stage I subjects of a lower risk group, which are not always given adjuvant treatment. For stage I subjects of a higher risk group and stage II subjects, the key question may instead be the intensity of the adjuvant treatment or whether or not to apply a combination treatment (e.g. radiation therapy + chemotherapy).

In addition, ASRGL1, as a marker for which a certain level of expression is correlated with a certain pattern of disease progression, may be included in a panel for making predictions or prognoses or for the selection of a treatment regimen.

In the method of the first aspect, it is determined whether an endometrial cancer subject belongs to a first or a second group, wherein subjects of the first group generally have a better prognosis than subjects of the second group. The division of endometrial cancer subjects into the two groups is determined by comparing sample values from the subjects with a reference value. Various reference values may be employed to discriminate between subjects that generally survived for a comparatively long period and subjects that generally survived for a comparatively short period. The reference value is thus the determinant for the size of the respective groups; the higher the reference value, the fewer the subjects in the first group and the lower the likelihood that a tested subject belongs to the first group. As the prognosis generally improves when the sample value increases, a relatively high reference value may in some instances be selected to identify subjects with a particularly good prognosis. Likewise, a relatively low reference value may in some instances be selected to identify subjects with a particularly poor prognosis. Guided by the present disclosure, the person skilled in the art may select relevant reference values without undue burden.

The first and the second group may consist exclusively of subjects having endometrial cancers of the same or similar grade, stage and/or type as the tested subject.

When the first and the second group consist exclusively of subjects having endometrial cancers of the same stage as the tested subject, the prognosis is a stage-independent prognosis (see e.g. Fig. 5 and 8).

When the first and the second group consist exclusively of subjects having endometrial cancers of the same grade as the tested subject, the prognosis is a grade-independent prognosis (see e.g. Fig. 3 and 4).

A stage-independent prognosis is particularly interesting as it provides information beyond what is available from the traditional staging and a grade-independent prognosis is particularly interesting as it provides information beyond what is available from the traditional grading.

Further, the groups may consist only of subjects having the same or similar age, race, sex, genetic characteristics and/or medical status or history.

Consequently, a physician may use the method according to the first aspect to obtain additional information regarding the prognosis of an endometrial cancer subject, which in turn may help in making informed decisions regarding following actions.

The prognosis of the tested subject may also be determined relative to a reference prognosis. Accordingly, as a first configuration of the first aspect, there is provided a method for determining a prognosis for a mammalian subject having an endometrial cancer, comprising the steps of:
a) evaluating an amount of ASRGL1 in at least part of a sample earlier obtained from the subject and determining a sample value corresponding to the evaluated amount;
b) comparing said sample value with a reference value associated with a reference prognosis; and if said sample value is higher than said reference value,
c1) concluding that the prognosis for said subject is better than said reference prognosis; and/or
   if said sample value is lower than or equal to said reference value,
c2) concluding that the prognosis for said subject is worse than or equal to said reference prognosis.

However closely related and covered by the same concept, c1) and c2) provide two alternative conclusions.

In the present disclosure, different ASRGL1 values (sample values) corresponding to various prognoses are presented. Typically, a high sample value is associated with a better prognosis than a low sample value.

The "reference prognosis" of the first configuration of the first aspect may be based on a previously established prognosis, e.g., obtained by an examination of a relevant population of subjects. Such reference population may be selected to match the tested subject's age, sex, race, endometrial cancer stage, grade and/or type and/or medical status and history. Further, a prognosis may be adapted to a background risk in the general population, a statistical prognosis/risk or an assumption based on an examination of the subject. Such examination may also comprise the subject's age, sex, race, endometrial cancer stage, endometrial cancer type and/or medical status and history. Thus, a physician may for example adapt the reference prognosis to the subject's endometrial cancer history, the type, grade and/or stage of the tumor, the morphology of the tumor, the location of the tumor, the presence and spread of metastases and/or further cancer characteristics.

The inventive concept of the present disclosure may also form the basis for a decision to refrain from a certain treatment regimen. For example, the prognoses for subjects showing high ASRGL1 levels are generally better than those for subjects showing low ASRGL1 levels, as shown in the attached figures. Provided with the teachings of the present disclosure, a physician may conclude that a comprehensive treatment regimen is not motivated and that a less aggressive treatment regimen is sufficient when the ASRGL1 level is high. Also, the decision may be to refrain from any adjuvant treatment in case of a high ASRGL1 level.

Thus, as a second configuration of the first aspect, there is provided a method for determining whether a subject having an endometrial cancer is not in need of an endometrial cancer treatment regimen, comprising the steps of:
a) evaluating an amount of ASRGL1 in at least part of a sample earlier obtained from the subject and determining a sample value corresponding to the evaluated amount;
b) comparing said sample value with a predetermined reference value;
   and
   if said sample value is higher than said reference value,
c) concluding that said subject is not in need of the endometrial cancer treatment regimen.

Further, as a third configuration of the first aspect, there is provided a non-treatment strategy method for a subject having an endometrial cancer, comprising the steps of:
a) evaluating an amount of ASRGL1 in at least part of a sample earlier obtained from the subject and determining a sample value corresponding to the evaluated amount;
b) comparing said sample value with a predetermined reference value;
   and
   if said sample value is higher than said reference value,
c) refraining from treating said subject with an endometrial cancer treatment regimen.

For example, step c) of the third configuration may be a refraining from the treatment regimen during at least one week from the completion of steps a) - b), such as at least one month from the completion of steps a) - b), such as at least three months from the completion of steps a) - b), such as at least six months from the completion of steps a) - b), such as at least one year from the completion of steps a) - b), such as at least two years from the completion of steps a) - b).

Alternatively, the refraining of step c) may be a refraining from treatment until the next time the method is performed or until a recurrence of an endometrial cancer.

In general, when deciding on a suitable treatment strategy for a patient having endometrial cancer, the physician responsible for the treatment may take several parameters into account, such as the result of an immunohistochemical evaluation, patient age, tumor type, stage and grade, general condition and medical history, such as endometrial cancer history. To be guided in the decision, the physician may perform an ASRGL1 test, or order an ASRGL1 test performed, according to the first aspect. Further, the physician may assign to someone else, such as a lab worker, to perform step a), and optionally step b), while performing step c), and optionally b), himself.

In the context of the present disclosure, "prognosis" refers to the prediction of the course or outcome of a disease and its treatment. For example, prognosis may also refer to a determination of chance of survival or recovery from a disease, as well as to a prediction of the expected survival time of a subject. A prognosis may further be represented by a single value or a range of values.

In embodiments of the present disclosure, the prognosis may be a probability of survival, and there are several ways to measure "survival". The survival of the present disclosure may for example be overall survival, progression free survival or disease specific survival (see the figures). It may also be a recurrence free survival. Further, the "survival" may be measured over different periods, such as five, ten or 15 years. Accordingly, the survival may be a five-year, ten-year or 15-year survival. The skilled person understands that when a reference prognosis is employed, it is of the same type as the prognosis for the subject.

As a fourth configuration of the first aspect, there is provided a method for determining whether a first or a second number of lymph nodes shall be removed from a mammalian subject having an endometrial cancer, wherein the first number is higher than the second number, comprising the steps of:
c) evaluating an amount of ASRGL1 in at least part of a sample earlier obtained from the subject and determining a sample value corresponding to the evaluated amount;
d) comparing said sample value with a predetermined reference value;
   and
   if said sample value is higher than said reference value,
c1) concluding that the second number of lymph nodes shall be removed; and
if said sample value is lower than or equal to said reference value, c2) concluding that the first number of lymph nodes shall be removed.

The sample of the fourth aspect is preferably a tissue sample obtained from a preoperative biopsy.

The removal of the lymph nodes (lymphadenectomy) referred to in the fourth configuration is preferably carried out in combination with hysterectomy.

For example, the removal of the first number of lymph nodes may be pelvic and periaortic lymphadenectomy while the removal or the second number of lymph nodes is pelvic lymphadenectomy only.

The inventive concept of the present disclosure may also form the basis for applying various treatment regimens.

For example, the prognosis for subjects showing low ASRGL1 levels is generally worse than those for subjects showing high ASRGL1 levels, as shown in the attached figures. Accordingly, a physician may consider the prognosis of an ASRGL1 low subject as being so poor that a certain treatment regimen is appropriate. The present disclosure may thus provide for accurate treatment of a previously undertreated group.

As a second aspect of the present disclosure, there is provided a method of treatment of a subject having an endometrial cancer, comprising the steps of:
a) evaluating an amount of ASRGL1 in at least part of a sample earlier obtained from the subject and determining a sample value corresponding to the evaluated amount;
b) comparing said sample value with a predetermined reference value;
   and
   if said sample value is lower than or equal to said reference value, c) treating said subject with an endometrial cancer treatment regimen. In embodiments of the second aspect, the method may further comprise the step of:
d) refraining from treating said subject with the endometrial cancer treatment regimen if said sample value is higher than said reference value.

The method of the second aspect may also comprise the step of:
d') treating said subject with another endometrial cancer treatment regimen, which is less comprehensive than the treatment regimen of step c), if said sample value is higher than said reference value.

The ASRGL1 level may be determined in a sample taken from the subject before surgery. Thus, as further discussed above, a method according to the present disclosure may be employed for determining the extent of the surgery. Accordingly, the endometrial cancer treatment regimen of the present disclosure may comprise surgery. For example, it may comprise hysterectomy in combination with lymphadenectomy. Thus, the result of the method of the third configuration of the first aspect may be to refrain from the hysterectomy in combination with lymphadenectomy when the sample value is higher than the reference value. Likewise, step c) of the second configuration of the first aspect may be concluding that the subject is not in need of hysterectomy in combination with lymphadenectomy.

It follows from the above that in the method of treatment (second aspect), hysterectomy in combination with lymphadenectomy may be applied in step c) when the sample value is lower than or equal to the reference value. If however the sample value is higher than the reference value, the hysterectomy may be performed without lymphadenectomy. Thus, the less comprehensive treatment regimen of step d') may be hysterectomy only.

Alternatively, step c) may comprise removal of a first number of lymph nodes when the sample value is lower than or equal to the reference value. If however the sample value is higher than the reference value, a second number of lymph nodes may be removed, wherein the second number is lower than the first number. Thus, the less comprehensive treatment regimen of step d') may be hysterectomy in combination with removal of the second number of lymph nodes.

As in the fourth configuration of the first aspect, the removal of the first number of lymph nods may be pelvic and periaortic lymphadenectomy while the removal of the second number of lymph nodes is pelvic lymphadenectomy only.

The question of whether to perform lymphadenectomy in addition to hysterectomy is particularly relevant for subject preoperatively stratified into the intermediate or high risk group. Thus, when the endometrial cancer treatment regimen of the present disclosure comprises hysterectomy in combination with lymphadenectomy, the subject's cancer is preferably grade 3 or has more than 50 % myometrial infiltration.

Independent of if lymphadenectomy is performed, the hysterectomy of the present disclosure is normally combined with bilateral salpingo-oophorectomy.

After surgery, the stratification is normally reevaluated. The main clinical postoperative questions are if an adjuvant treatment shall be applied and in such case, what kind of adjuvant treatment to apply.

Thus, the endometrial cancer treatment regimen of the present disclosure may comprise or consist of adjuvant treatment.

The question of whether to apply any adjuvant treatment at all is particularly relevant for stage I subjects postoperatively assigned to the low or intermediate risk group (grade 1 or 2 or less than 50 % myometrial infiltration). If it is decided to apply an adjuvant treatment to such subjects, it is normally radiation therapy, such as vaginal brachytherapy.

For such subjects, the result of the method of the third configuration of the first aspect may thus be to refrain from all adjuvant therapy when the sample value is higher than the reference value. Likewise, step c) of the second configuration of the first aspect may be concluding that the subject is not in need of any adjuvant treatment for such subjects.

It follows from the above that in the method of treatment (second aspect), an adjuvant treatment, such as a radiation therapy, may be applied in step c) when the sample value is lower than or equal to the reference value. If however the sample value is higher than the reference value, step d) of the second aspect may thus be to refrain from any adjuvant treatment. Alternatively, a radiation therapy of a first intensity may be applied in step c) (i.e. when the ASRGL1 level is low) and a radiation therapy of a second intensity may be applied in step d') (i.e. when the ASRGL1 level is high), wherein the first intensity is higher than the second intensity. For example, the total dose of the radiation therapy may be higher according to the first intensity than according to the second intensity. Radiation therapy is normally fractionized. Thus, the dose per fraction may be higher according to the first aspect than according to the second aspect. Also, the radiation treatments/events may be higher in number, more frequent or applied during a longer period according to the first aspect than according to the second aspect.

For stage I subjects assigned to the high risk group or for stage II subjects, the relevant clinical question may instead be if an adjuvant combination therapy is needed or if an adjuvant monotherapy, such as vaginal brachytherapy, is sufficient. Here, the combination treatment is normally a combination of a radiation therapy and a chemotherapy or a combination of one type of radiation therapy (such as brachytherapy) with another type of radiation therapy (such as external pelvic, or periaortic, radiation therapy in cases where no lymphadenectomy has been performed).

The chemotherapy of the present disclosure may for example be application of paclitaxel, preferably in combination with carboplatin. Alternatively, the chemotherapy may be doxorubicin, liposomal doxorubicin or epirubicin. The chemotherapy may also be application of paclitaxel, possibly in combination with carboplatin, followed by doxorubicin, liposomal doxorubicin or epirubicin. The carboplatin of the chemotherapy of the present disclosure may by replaced by cisplatin. The chemotherapy may also comprise cyclofosfamide or ifosfamide. As another example, the chemotherapy may comprise hormonal therapy, such as application of a progestational agent (hydroxyprogesterone, medroxyprogesterone or megestrol), tamoxifen or progestin. As understood by the skilled person, a hormonal treatment is normally only considered for hormone receptor positive tumors.

For the subject having a stage I tumor of grade 3 with more than 50 % myometrial infiltration or a stage II tumor, the result of the method of the third configuration of the first aspect may thus be to refrain from the adjuvant combination treatment when the sample value is higher than the reference value. Likewise, step c) of the second configuration of the first aspect may be concluding that the subject is not in need of the combination treatment for such a subject.

It follows from the above that in the method of treatment (second aspect), the combination treatment may be applied in step c) when the sample value is lower than or equal to the reference value. If however the sample value is higher than the reference value, step d') of the second aspect may be to apply a monotherapy, such as a radiation therapy, such as vaginal brachytherapy.

Alternatively, the endometrial treatment regimen of step c) of the second aspect comprises a chemotherapy of a first intensity and the less comprehensive chemotherapy of step d) of the second aspect comprises a chemotherapy of a second intensity, wherein the first intensity is higher than the second intensity.

The level of intensity of a chemotherapy may for example be measured as the average daily or weekly dose of a therapeutic agent given to the subject. A chemotherapy of the first intensity may thus be applied more frequently or in higher individual doses than a chemotherapy of the second intensity. The chemotherapy of the first intensity may also comprise application of a more aggressive therapeutic agent than the chemotherapy of the second intensity. Alternatively, the first intensity may comprise application of more therapeutic agents than the chemotherapy of the second intensity. Thus, the chemotherapy of the first intensity may be a combined application of two chemotherapeutic agents, such as paclitaxel and carboplatin, while the chemotherapy of the second intensity is an application of a single chemotherapeutic agent, such as paclitaxel only. Also, the chemotherapy of the first intensity may be a separate, sequential or simultaneous application of three chemotherapeutic agents, while the chemotherapy of the second intensity is a separate, sequential or simultaneous application of three chemotherapeutic agents. Yet another possibility is that the chemotherapy of the first intensity is applied for a longer period than the chemotherapy of the second intensity.

As understood from the above reasoning, it may be particularly difficult to find the most appropriate treatment for stage I and II subjects, which means that a biomarker is particularly relevant for such subjects. The endometrial cancer of the present disclosure may thus be stage I or II. The prognostic relevance of ASRGL1 in stage I is shown in Fig. 5A, 8A and 8B. The prognostic relevance of ASRGL1 in stage II is shown in Fig. 5B.

The grade and the degree of myometrial infiltration may also influence the treatment decision, as discussed above. Thus, the endometrial cancer of the present disclosure may be grade 1, 2 or 3. In Fig. 4 and 5, ASRGL1 expression is shown to correlate with the prognosis of subjects having cancers of all three grades. The myometrial infiltration may be more or less than 50 %.

As understood from the discussion above, it is particularly relevant to obtain further prognostic information about grade 1 or 2 tumors with > 50 % myometrial infiltration. Further, the prognostic relevance of the ASRGL1 expression is particularly accentuated in this subgroup (see Fig. 7B and 8). Thus, in embodiments of the present disclosure, the endometrial cancer is grade 1 or 2 tumors with > 50 % myometrial infiltration.

The physician responsible for the treatment according to the second aspect may assign to someone else, such as a lab worker, to perform step a), and optionally step b), while performing step c), and optionally b), himself. Further, the results of steps a) and b) may be at hand when the method of treatment according to the second aspect is initiated.

The method of treatment may also be limited to the decision-making and treatment. Thus, as a configuration of the second aspect, there is provided a method of treatment of a subject having an endometrial cancer, comprising:
α) comparing a sample value corresponding to a level of ASRGL1 in a sample from the subject with a reference value; and,
   if said sample value is lower than or equal to said reference value,
β) treating said subject with an endometrial cancer treatment regimen.

Numerous ways of obtaining a sample value corresponding to a level of ASRGL1 in a sample from a subject are described in the present disclosure. The endometrial cancer treatment regimen of β) may be selected according to the above.

Further, the skilled person should recognize that the usefulness of the aspects of the present disclosure is not limited to the quantification of any particular variant of the ASRGL1 protein or ASRGL1 mRNA present in the subject in question, as long as the protein is encoded by the relevant gene and presents the relevant pattern of expression. As a non-limiting example, the ASRGL1 protein may comprise a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the ASRGL1 protein may comprise, or consists of, a sequence selected from:
i) SEQ ID NO:2 or 3; and
ii) a sequence which is at least 85 % identical to SEQ ID NO:2. SEQ ID NO:2 and 3 are two isoforms of the ASRGL1 protein.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2 or 3.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acids may at the most be 85 % identical to a target sequence of 100 amino acids.

Regarding step a) of the methods of the present disclosure, an increase in the amount of ASRGL1 typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of ASRGL1 will not result in a decrease in the sample value in the context of the present disclosure.

However inconvenient, but in an equivalent fashion, the evaluated amounts may be inversely related to sample values if the qualification between step b) and c) is inverted. For example, in the first aspect the qualification between step b) and c) is inverted if the phrase "if the sample value is higher than the reference value" (before c1)) is replaced with "if the sample value is lower than the reference value" and the phrase "if the sample value is lower than or equal to the reference value" (before c2)) is replaced with "if the sample value is higher than or equal to the reference value".

Further, in the context of the methods of the present disclosure, "earlier obtained" refers to obtained before the method is performed. Consequently, if a sample earlier obtained from a subject is used in a method, the method does not involve obtaining the sample from the subject, i.e., the sample was previously obtained from the subject in a step separate from the method.

The methods and uses of the present disclosure, except the methods of treatment, may unless otherwise stated or indicated be carried out entirely *ex vivo.*

Further, in the context of the present disclosure, "a mammalian subject having an endometrial cancer" refers to a mammalian subject having a primary endometrial tumor or a mammalian subject which has had a primary endometrial tumor removed, wherein the removal of the tumor refers to eradicating the tumor by any appropriate type of surgery or therapy. In the method and use aspects of the present disclosure, "a mammalian subject having an endometrial cancer" also includes the cases wherein the mammalian subject is suspected of having an endometrial cancer at the time of the use or the performance of the method and the endometrial cancer diagnosis is established later.

Further, in the context of the present disclosure, the "predetermined reference value" refers to a predetermined value found to be relevant for making decisions or drawing conclusions regarding the prognosis or a suitable treatment strategy for the subject.

Also, in the context of the present disclosure, a reference value being "associated" with a reference prognosis refers to the reference value being assigned a corresponding reference prognosis, based on empirical data and/or clinically relevant assumptions. For example, the reference value may be the average ASRGL1 value in a relevant group of subjects and the reference prognosis may be an average survival in the same group. Further, the reference value does not have to be assigned to a reference prognosis directly derived from prognosis data of a group of subjects exhibiting the reference value. The reference prognosis may for example correspond to the prognosis for subjects exhibiting the reference value or lower. That is, if the reference value is 1 on a scale from 0 to 2, the reference prognosis may be the prognosis of the subjects exhibiting the values 0 or 1. Consequently, the reference prognosis may also be adapted to the nature of the available data. As further discussed above, the reference prognosis may be further adapted to other parameters as well.

Step a) of the methods of the above aspects involve evaluating an amount of ASRGL1 present in at least part of the sample, and determining a sample value corresponding to the amount. The "at least part of the sample" refers to a relevant part or relevant parts of the sample for establishing the prognosis or drawing conclusions regarding suitable treatments. The person skilled in the art understands which part or parts that are relevant under the circumstances present when performing the method. For example, if evaluating a sample comprising cells, the skilled person may only consider the tumor cells, or only the cytoplasms of tumor cells, of the sample.

Further, in step a) an amount is evaluated and a sample value corresponding to the amount is determined. Consequently, an exact measurement of the amount of ASRGL1 is not required for obtaining the sample value. For example, the amount of ASRGL1 may be evaluated by visual inspection of a prepared and stained tissue sample and the sample value may then be categorized as for example high or low based on the evaluated amount.

In embodiments of the methods of the above aspects, the sample may be a body fluid sample. For example, the body fluid sample may be selected from the group consisting of blood, plasma, serum, cerebral fluid, urine, lymph, seminal fluid and exudate. Alternatively, the sample may be a cytology sample or a stool sample.

The level of ASRGL1 is preferably measured in cells or cell-derived material. Thus, the body fluid, cytology or stool sample may for example comprise cells, such as tumor cells, such as endometrial cancer tumor cells from said subject.

In further embodiments of the methods of the above aspects, the sample may be a tissue sample, such as a tumor tissue sample.

The tumor tissue sample is preferably an endometrial tumor tissue sample, such as a tumor tissue sample from the endometrial glands.

The tissue sample may for example derive from a biopsy, such as a preoperative biopsy. In such case, the ASRGL1 level of the tissue sample may be used to determine the type of surgery to perform. For example, a preoperative tissue sample may be used when it is determined if hysterectomy in combination with lymphadenectomy shall be performed or if only hysterectomy is sufficient. Alternatively, the preoperative tissue sample may be used when to determine if the hysterectomy shall be combined with the removal of a first (higher) or second (lower) number of lymph nodes.

The tissue sample may also be derived from a surgically removed specimen. When determining the adjuvant treatment strategy, it is preferred to use such as tissue sample.

The inventors have noted that cytoplasmic expression of ASRGL1 is particularly relevant for determining an endometrial cancer prognosis (see the Example below). The evaluation of step a) may thus be limited to the cytoplasms of cells, such as tumor cells, of said sample.

Consequently, when a tissue sample is examined, only the cytoplasms of tumor cells may be taken into consideration. Such examination may for example be aided by immunohistochemical staining.

However, the inventors have noted that ASRGL1 may also be expressed in the nuclei of tumor cells. Thus, in embodiments of the present disclosure, nuclear expression may be considered, e.g. in step a) of the methods.

When performing the methods according to the above aspects, it may be convenient to use zero as the reference value, because in such case, it has only to be established in step a) whether ASRGL1 is present in the sample or not. The figures show that a value of zero is a working cut-off value for establishing two subgroups of significantly different prognoses.

Thus, in embodiments of the methods of the above aspects, the sample value of step a) may be either 1, corresponding to detectable ASRGL1 in tumor cells of the sample, or 0, corresponding to no detectable ASRGL1 in tumor cells of the sample. Consequently, in such embodiments, the evaluation of the sample is digital: ASRGL1 is considered to be either present or not. In the context of the present disclosure, "no detectable ASRGL1" refers to an amount of ASRGL1 that is so small that it is not, during normal operational circumstances, detectable by a person or an apparatus performing the step a). The "normal operational circumstances" refer to the laboratory methods and techniques a person skilled in the art would find appropriate for performing the methods of the present disclosure.

A sample value of ASRGL1 being higher than the reference value, or a subject from which such sample value is obtained, is sometimes referred to herein as being "ASRGL1 high". Further, a sample value of ASRGL1 being lower than, or equal to, the reference value, or a subject from which such sample value is obtained, is sometimes referred to herein as being "ASRGL1 low".

In the context of the present disclosure, the terms "sample value" and "reference value" are to be interpreted broadly. The quantification of ASRGL1 to obtain these values may be done via automatic means, via a scoring system based on visual or microscopic inspection of samples, or via combinations thereof. However, it is also possible for a skilled person, such as a person skilled in the art of histopathology, to determine the sample and/or reference value by inspection, e.g., of tissue slides that have been prepared and stained for ASRGL1 protein expression.

Determining that the sample value is higher than the reference value may thus be determining, upon visual or microscopic inspection, that a sample tissue slide is more densely stained and/or exhibit a larger fraction of stained cells than a reference tissue slide. The sample value may also be compared to a reference value given by a literal reference, such as a reference value described in wording or by a reference picture. Consequently, the sample and/or reference values may in some cases be mental values that the skilled person envisages upon inspection and comparison.

For example, the skilled person may categorize a sample as being ASRGL1 protein high or low, wherein the sample is categorized as high if it contains more ASRGL1 protein than a previously inspected reference sample and low if it contains less or equally much. Such evaluation may be assisted by staining the sample, and, if necessary, a reference sample, with a staining solution comprising e.g., antibodies selective for ASRGL1 protein.

One or more of the steps of the methods of the present disclosure may be implemented in an apparatus. For example, step a) and optionally step b) may be performed in an automatic analysis apparatus, and such an apparatus may be based on a platform adapted for immunohistochemical analysis. As an example, one or more tumor tissue sample(s) from the subject in question may be prepared for imunohistochemical analysis manually and then loaded into the automatic analysis apparatus, which gives the sample value of step a) and optionally also performs the comparison with the reference value of step b). The operator performing the analysis, the physician ordering the analysis or the apparatus itself may then draw the conclusion of step c). Consequently, software adapted for drawing the conclusion of step c) may be implemented on the apparatus.

A reference value, which is relevant for establishing a prognosis or making a treatment decision regarding endometrial cancer subjects, for use as comparison with the sample value from the subject, may be provided in various ways. With the knowledge of the teachings of the present disclosure, the skilled artisan can, without undue burden, provide relevant reference values for performing the methods of the present disclosure.

The person performing the methods of the above aspects may, for example, adapt the reference value to desired information. For example, the reference value may be adapted to yield the most significant prognostic information, e.g., the largest separation between the ASRGL1 high survival curve and the ASRGL1 low survival curve (see the figures), which corresponds to the largest difference in survival between the first and the second group of the first aspect. Alternatively, the reference value may be selected such that a group of subjects having a particularly poor prognosis is singled out.

In embodiments of the methods of the above aspects, the reference value may correspond to the amount of ASRGL1 expression in a healthy tissue, such as healthy endometrial tissue, or stroma tissue of the subject of the method. As another example, the reference value may be provided by the amount of ASRGL1 expression measured in a standard sample of normal tissue from another, comparable subject. As another example, the reference value may be provided by the amount of ASRGL1 expression measured in a reference sample comprising tumor cells, such as a reference sample of tumor tissue, e.g., endometrial tumor tissue. The amount of protein expression of the reference sample may preferably be previously established.

Further, the reference value may for example be provided by the amount of ASRGL1 expression measured in a reference sample comprising cell lines, such as cancer cell lines, expressing a predetermined, or controlled, amount of ASRGL1. The person skilled in the art understands how to provide such cell lines, for example guided by the disclosure of Rhodes et al. (2006) The biomedical scientist, p 515-520.

Consequently, the reference value may be provided by the amount of ASRGL1 measured in a reference sample comprising cells expressing a predetermined amount of ASRGL1. Accordingly, in embodiments of the methods of the present disclosure, the reference value may be a predetermined value corresponding to the amount of ASRGL1 expression in a reference sample.

However, the amount of ASRGL1 protein in the reference sample does not have to directly correspond to the reference value (this is further discussed below). The reference sample may also provide an amount of ASRGL1 protein that helps a person performing the method to assess various reference values. The reference sample(s) may thus help in creating a mental image of the reference value by providing a "positive" reference value and/or a "negative" reference value. For example, there may be provided one reference sample having a positive ASRGL1 expression in the cytoplasms of the tumor cells (a positive reference) and another reference sample having absent ASRGL1 expression in the cytoplasms of the tumor cells (a negative reference). Here, the latter reference sample may also provide the actual reference value.

One alternative for the quantification of ASRGL1 protein in a sample, such as the sample earlier obtained from the subject or the reference sample, is the determination of the fraction of cells in the sample that exhibit ASRGL1 protein expression over a certain level. The fraction may for example be: a "cellular fraction", wherein the ASRGL1 protein expression of the whole cells is taken into account; or a "cytoplasmic fraction", wherein the ASRGL1 protein expression of only the cytoplasms of the cells is taken into account. The cellular, or cytoplasmic fraction may for example be classified as < 2 %, 2 - 10 %, 11 - 25 %, 26 - 50 %, 51 - 75 % or > 75 % immunoreactive cells of the relevant cell population. The "cytoplasmic fraction" corresponds to the percentage of relevant cells in a sample that exhibits a positive staining in the cytoplasm, wherein a medium or distinct and strong immunoreactivity in the cytoplasm is considered positive and no or faint immunoreactivity in the cytoplasm is considered negative. The person skilled in the art of pathology understands which cells that are relevant under the conditions present when performing the method and may determine a cellular or cytoplasmic fraction based on his general knowledge and the teachings of the present disclosure. The relevant cells may for example be tumor cells.

Another alternative for the quantification of ASRGL1 protein expression in a sample, such as the sample earlier obtained from the subject or the reference sample, is the determination of the overall staining intensity of the sample. The intensity may for example be: a "cellular intensity", wherein the ASRGL1 protein expression of the whole cells is taken into account; or a "cytoplasmic intensity", wherein the ASRGL1 protein expression of only the cytoplasms of the cells is taken into account. Outcome of a cytoplasmic intensity determination may be classified as: absent = no overall immunoreactivity in the cytoplasms of relevant cells of the sample, weak = faint overall immunoreactivity in the cytoplasms of relevant cells of the sample, moderate = medium overall immunoreactivity in the cytoplasms of relevant cells of the sample, or strong = distinct and strong overall immunoreactivity in the cytoplasms of relevant cells of the sample. The person skilled in the art understands which cells that are relevant under the conditions present when performing the method and may determine a cellular or cytoplasmic intensity based on his general knowledge and the teachings of the present disclosure. The relevant cells may for example be tumor cells.

The inventors have found that cytoplasmic expression of ASRGL1 protein is particularly relevant for establishing prognoses.

Thus, in embodiments of the methods of the above aspects, the reference value may be a cytoplasmic fraction, a cytoplasmic intensity or a function thereof. Accordingly, the sample value may be a cytoplasmic fraction, a cytoplasmic intensity or a function thereof.

An example of such a function is a staining score (SS), which is the product of the cytoplasmic fraction (CF) value and the cytoplasmic intensity (CI) value determined according to the table below.

| Cl | Cl value | CF (%) | CF value |
|---|---|---|---|
| absent | 1 | <2 | 1 |
| weak | 2 | 2-10 | 2 |
| moderate | 3 | 11-25 | 3 |
| strong | 4 | 26-50 | 4 |
| | | 51-75 | 5 |
| | | >75 | 6 |

The SS may thus range from 1 to 24.

The person skilled in the art realizes that various combinations or functions of fractions and intensities or other values may be used as the reference value within the framework of the present disclosure. Consequently, the reference value may involve two, and possibly even more, criteria.

As can be seen in the figures, relatively low reference values ("cut-off values") establish two groups of subjects having significantly different prognoses (see Fig. 1, 2, 4, 6, 7 and 9). Thus, the reference value may be an absent or weak cytoplasmic intensity, a cytoplasmic fraction of 10 % or lower or a staining score of 1 or 2. For example, it may be an absent cytoplasmic intensity (Fig. 2A, 4, 6A and 7A), a cytoplasmic fraction of 1 % or lower (Fig. 2B, 6B, 7B) or a staining score of 1 (Fig. 9).

However, a relatively high reference value also establishes two groups of subjects having significantly different prognoses (see Fig. 1, 3, 5 and 8). Thus, the reference value may be a strong cytoplasmic intensity or a cytoplasmic fraction of at least 50 %, such as at least 70 %, such as about 75 % (Fig. 3, 5 and 8).

In general, the selection of the reference value may depend on the staining procedure, e.g., on the employed anti-ASRGL1 antibody and on the staining reagents.

The selection of the reference value may also depend on if it is relevant to identify a particularly poor prognosis (which would motivate a more aggressive treatment) or a particularly good prognosis (which would motivate a less aggressive treatment or no adjuvant treatment). The particularly poor prognosis is generally identified if the sample value is below or equal to one of the relatively low reference values (see above) and the particularly good prognosis is generally identified if the sample value is above one of the relatively high reference values (see above).

Guided by the present disclosure, a person skilled in the art, e.g. a pathologist, understands how to perform the evaluation yielding a fraction, such as a cellular or cytoplasmic fraction, or an intensity, such as a cellular or cytoplasmic intensity. For example, the skilled artisan may use a reference sample comprising a predetermined amount of ASRGL1 protein for establishing the appearance of a certain fraction or intensity.

However, a reference sample may not only be used for the provision of the actual reference value, but also for the provision of an example of a sample having an amount of ASRGL1 protein, that is higher than the amount corresponding to the reference value. As an example, in histochemical staining, such as in immunohistochemical staining, the skilled artisan may use a reference sample for establishing the appearance of a stained sample having a high amount of ASRGL1 protein. Such a reference sample is thus a positive reference. Subsequently, the skilled artisan may assess the appearances of samples having lower amounts of ASRGL1 protein, such as the appearance of a sample with an amount of ASRGL1 protein corresponding to the reference value. In other words, the skilled artisan may use a reference sample to create a mental image of a reference value corresponding to an amount of ASRGL1 protein which is lower than that of the reference sample. Alternatively, or as a complement, in such assessments, the skilled artisan may use another reference sample having a low amount of ASRGL1 protein, or lacking detectable ASRGL1 protein, for establishing the appearance of such sample, e.g., as a "negative reference".

For example, if a cytoplasmic fraction of 10 % is used as the reference value, two reference samples may be employed: a first reference sample having no detectable ASRGL1 protein; and a second reference sample having an amount of ASRGL1 protein corresponding to a cytoplasmic fraction of at least 75 %, which is higher than the reference value.

Consequently, in the evaluation, the skilled artisan may use a reference sample for establishing the appearance of a sample with a high amount of ASRGL1 protein. Such reference sample may be a sample comprising tissue expressing a high amount of ASRGL1 protein, such as a sample comprising endometrial tumor tissue having a pre-established high expression of ASRGL1 protein.

As mentioned above, cell lines expressing a controlled amount of ASRGL1 protein may be used as the reference, in particular as a positive reference.

One or more pictures may also be provided as the "reference sample". For example, such a picture may show an example of a tumor tissue slide stained with a certain antibody during certain conditions exhibiting a certain cytoplasmic intensity and/or fraction. The above discussion about the "reference sample" applies *mutatis mutandis* to pictures.

In some embodiments, step a) of the methods of the above aspects may comprise:
obtaining biological material from the subject, excising or selecting a relevant part of the biological material to obtain said sample and optionally arranging the sample on a solid phase to facilitate the evaluation of step a). Step a) may thus, as an example, comprise obtaining endometrial tissue material from the subject, optionally fixating the tissue material in paraffin or formalin, histo-processing the tissue material to obtain a section which constitute said sample and optionally mounting said sample on a transparent slide, such as a glass slide, for microscopy.

In embodiments of the methods of the aspects above, the ASRGL1 protein may be detected and/or quantified through the application to the sample of a detectable and/or quantifiable affinity ligand, which is capable of selective interaction with the ASRGL1 protein. The application of the affinity ligand is performed under conditions that enable binding of the affinity ligand to ASRGL1 protein in the sample.

In more detail, step a) of some embodiments of the methods of the above aspects may comprise:
a1) applying to said sample a quantifiable affinity ligand capable of selective interaction with the ASRGL1 protein to be evaluated, said application being performed under conditions that enable binding of said affinity ligand to ASRGL1 protein present in said sample;
a2) removing non-bound affinity ligand; and
a3) quantifying the affinity ligand remaining in association with said sample to evaluate said amount.

"Affinity ligand remaining in association with the sample" refers to affinity ligand which was not removed in step a2), e.g., the affinity ligand bound to the sample. Here, the binding may for example be the interaction between antibody and antigen.

However, the removal of non-bound affinity ligand according to a2), e.g. the washing, is not always necessary. Thus, in some embodiments of the methods of the aspects above, step a) may comprise:
a1) applying to said sample a quantifiable affinity ligand capable of selective interaction with the ASRGL1 protein to be evaluated, said application being performed under conditions that enable binding of said affinity ligand to ASRGL1 protein present in said sample;
aII) quantifying the affinity bound to said sample to evaluate said amount.

In the context of the present disclosure, "specific" or "selective" interaction of e.g., an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity/selectivity does not necessarily require high affinity. Molecules with low affinity (in the molar range) for its counterpart have been shown to be as selective/specific as molecules with much higher affinity. In the case of the present disclosure, a specific or selective interaction refers to the extent to which a particular method can be used to determine the presence and/or amount of a specific protein, the target protein, under given conditions in the presence of other proteins in a biological sample, such as a tissue sample or a fluid sample of a naturally occurring or processed biological fluid. In other words, specificity or selectivity is the capacity to distinguish between related proteins. For example, the specificity or selectivity of an antibody may be determined using a protein array set-up, a suspension bead array and a multiplexed competition assay, respectively (see e.g. Examples, section 2 of WO2011/051288). Specificity and selectivity determinations are also described in Nilsson P et al. (2005) Proteomics 5:4327-4337.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below for the sake of illustration.

Thus, in embodiments of the present disclosure, the affinity ligand may be selected from the group consisting of antibodies, fragments thereof and derivatives thereof. The affinity ligand may thus be based on an immunoglobulin scaffold. The antibodies and the fragments or derivatives thereof are normally isolated. Also, they may be antigen purified. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. the ASRGL1 protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

SEQ ID NO:1 was designed for immunizations, e.g., designed to lack transmembrane regions to ensure efficient expression in *E*. *coli,* and to lack any signal peptide, since those are cleaved off in the mature protein. Consequently, an antibody or fragment or derivative thereof according to the present disclosure may for example be one that is obtainable by a process comprising a step of immunizing an animal, such as a rabbit, with a protein whose amino acid sequence comprises, preferably consists of, the sequence SEQ ID NO:1. For example, the immunization process may comprise primary immunization with the protein or peptide in Freund's complete adjuvant. Also, the immunization process may further comprise boosting at least two times, in intervals of 2-6 weeks, with the protein or peptide in Freund's incomplete adjuvant. Processes for the production of antibodies or fragments or derivatives thereof against a given target are known in the art.

In the context of the present disclosure, an "antigen purified antibody" is one or a population of polyclonal antibodies which has been affinity purified on its own antigen, thereby separating such antigen purified antibodies from other antiserum proteins and non-specific antibodies. This affinity purification results in antibodies that bind selectively to its antigen. In the case of the present disclosure, the polyclonal antisera are purified by a two-step immunoaffinity based protocol to obtain antigen purified antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

Polyclonal and monoclonal antibodies, as well as their fragments and derivatives, represent the traditional choice of affinity ligands in applications requiring selective biomolecular recognition, such as in the detection and/or quantification of ASRGL1 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific and/or selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against ASRGL1 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101(25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

The ASRGL1 protein fragment SEQ ID NO:1 was designed to consist of a unique sequence with low sequence identity with other human proteins and to minimize cross reactivity of generated affinity reagents. Consequently, in embodiments of the present disclosure, the affinity ligand may be capable of selective interaction with a polypeptide consisting of the sequence SEQ ID NO:1.

"The affinity ligand capable of selective interaction with a polypeptide consisting of the sequence SEQ ID NO:1" is capable of distinguishing a SEQ ID NO:1 fragment from a fragment consisting of another, non-overlapping, part of the ASRGL1 protein.

The detection and/or quantification of the affinity ligand capable of selective interaction with the ASRGL1 protein may be accomplished in any way known to the skilled person for detection and/or quantification of binding reagents in assays based on biological interactions. Accordingly, any affinity ligand described above may be used to quantitatively and/or qualitatively detect the presence of the ASRGL1 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with ASRGL1 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole), bioluminescent proteins (e.g., luciferin, luciferase), and haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its ASRGL1 protein target may involve visualizing techniques, such as light microscopy (e.g. combined with scanning) or immunofluoresence microscopy. Other methods may involve the detection via flow cytometry or luminometry.

A biological sample, such as a tumor tissue sample, which has been removed from the subject, may be used for detection and/or quantification of ASRGL1 protein. The biological sample may be an earlier obtained sample. If using an earlier obtained sample in a method, no steps of the method are practiced on the human or animal body. The affinity ligand may be applied to the biological sample for detection and/or quantification of the ASRGL1 protein. This procedure enables not only detection of ASRGL1 protein, but may in addition show the distribution and relative level of expression thereof. Thus, cytoplasmic protein expression may be distinguished from nuclear protein expression.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified for proper detection and/or quantification.

In embodiments of the methods of the above aspects, a biological sample may be immobilized onto a solid phase support or carrier, such as nitrocellulose or any other solid support matrix capable of immobilizing ASRGL1 protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, a primary affinity ligand specific to ASRGL1 protein may be applied, e.g., as described in the Examples below. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of a1) or al) may be detected using a secondary affinity ligand capable of recognizing the quantifiable affinity ligand. The quantification of a3) or aII) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the ASRGL1 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the ASRGL1 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize ASRGL1 protein by detection of radioactivity *in vivo* or *ex vivo.* Radionuclear scanning with e.g., gamma camera, magnetic resonance spectroscopy or emission tomography function for detection *in vivo* and *ex vivo,* while gamma/beta counters, scintillation counters and radiographies are also used *ex vivo.*

Methods for detecting and quantifying biomarkers on the mRNA level are well known within the art.

The ASRGL mRNA of the present disclosure may be represented by cDNA sequence SEQ ID NO:4 or SEQ ID NO:5. SEQ ID NO:4 and SEQ ID NO:5 include non-coding sequences.

In general, total cellular RNA may be purified from cells by homogenization in the presence of nucleic acid extraction buffer, followed by centrifugation. Nucleic acids are then precipitated, in order to remove DNA by treatment with DNase and precipitation. The RNA molecules are then separated by gel electrophoresis on agarose gels according to standard techniques, and transferred to nitrocellulose filters by, e.g., the so-called "Northern" blotting technique. The RNA is then immobilized on the filters by heating. Detection and quantification of specific RNA is accomplished using appropriately labeled DNA or RNA probes complementary to the RNA in question. See, for example, Molecular Cloning: A Laboratory Manual (Sambrook J.et al., (1989) 2nd edition, Cold Spring Harbor Laboratory Press). Methods for the preparation of labeled DNA and RNA probes, and the conditions for hybridization thereof to target nucleotide sequences, are described in Molecular Cloning: A Laboratory Manual (Sambrook J.et al., (1989) 2nd edition, Cold Spring Harbor Laboratory Press). For example, the nucleic acid probe may be labeled with, e.g., a radionuclide such as ³H, ³²P, ³³P, ¹⁴C, or ³⁵S; a heavy metal; or a ligand capable of functioning as a specific binding pair member for a labeled ligand (e.g., biotin, avidin, or an antibody), a fluorescent molecule, a chemi luminescent molecule, an enzyme, or the like.

Probes may be labeled to high specific activity by either the nick translation method (Rigby et al., (1977) J. Mol Biol, 113: 237-251), or by the random priming method (Fienberg, (1983) Anal. Biochem., 132: 6-13). The latter can be a method for synthesizing ³²P-labeled probes of high specific activity from RNA templates. For example, by replacing preexisting nucleotides with highly radioactive nucleotides according to the nick translation method, it is possible to prepare ³²P- labeled nucleic acid probes with a specific activity well in excess of 10 cpm/microgram. Autoradiographic detection of hybridization then can be performed by exposing hybridized filters to photographic film. Densitometric scanning of the photographic films exposed by the hybridized filters provides an accurate measurement of biomarker levels. Using another approach, biomarker levels can be quantified by computerized imaging systems, such as the Molecular Dynamics 400-B 2D Phosphorimager (Amersham Biosciences, Piscataway, NJ., USA).

Where radionuclide labeling of DNA or RNA probes is not practical, the random- primer method can be used to incorporate an analogue, for example, the dTTP analogue 5 -(N-(N- biotinyl-epsilon-aminocaproyl)-3-aminoallyl)deoxyuridine triphosphate, into the probe molecule. The biotinylated probe oligonucleotide can be detected by reaction with biotin-binding proteins, such as avidin, streptavidin, and antibodies (e.g., anti-biotin antibodies) coupled to fluorescent dyes or enzymes that produce color reactions.

In addition to Northern and other RNA blotting hybridization techniques, determining the levels of RNA transcript may be accomplished using the technique of in situ hybridization. This technique requires fewer cells than the Northern blotting technique, and involves depositing whole cells onto a microscope cover slip and probing the nucleic acid content of the cell with a solution containing radioactive or otherwise labeled nucleic acid (e.g., cDNA or RNA) probes. This technique is particularly well-suited for analyzing tissue biopsy samples from subjects.

The relative number of RNA transcripts in cells also can be determined by reverse transcription of RNA transcripts, followed by amplification of the reverse-transcribed transcripts by polymerase chain reaction (RT-PCR). The levels of RNA transcripts can be quantified in comparison with an internal standard, for example, the level of mRNA from a standard gene present in the same sample. The person skilled in the art is capable of selecting suitable genes for use as an internal standard. The methods for quantitative RT-PCR and variations thereof are within the skill in the art.

Any suitable primers can be used for the quantitative RT-PCR. Preferably, the primers are specific to ASRGL1. It is within the skill in the art to generate primers specific to ASRGL1. Primers can be of any suitable length, but are preferably between 19 and 23 (e.g., 19, 20, 21, 22, or 23) nucleotides. Ideally, amplicon length should be 50 to 150 (up to 250 may be necessary but then optimization of the thermal cycling protocol and reaction components may be necessary) bases for optimal PCR efficiency. Designing primers that generate a very long amplicon may lead to poor amplification efficiency. Information about primer design and optimal amplicon size may fo example be found at www.ambion.com.

In some instances, it may be desirable to use microchip technology to detect biomarker expression. The microchip can be fabricated by techniques known in the art. For example, probe oligonucleotides of an appropriate length, e.g., 40 nucleotides, are 5'-amine modified at position C6 and printed using commercially available microarray systems, e.g., the GENEMACHINE OmniGrid 100 Microarrayer and Amersham CODELINK activated slides. Labeled cDNA oligomer corresponding to the target RNAs is prepared by reverse transcribing the target RNA with labeled primer. Following first strand synthesis, the RNA/DNA hybrids are denatured to degrade the RNA templates. The labeled target cDNAs thus prepared are then hybridized to the microarray chip under hybridizing conditions, e.g., 6 times SSPE/30% formamide at 25 °C for 18 hours, followed by washing in 0.75 times TNT at 37 °C for 40 minutes. At positions on the array, where the immobilized probe DNA recognizes a complementary target cDNA in the sample, hybridization occurs. The labeled target cDNA marks the exact position on the array where binding occurs, thereby allowing automatic detection and quantification. The output consists of a list of hybridization events, which indicate the relative abundance of specific cDNA sequences, and therefore the relative abundance of the corresponding complementary biomarker, in the subject sample. According to one embodiment, the labeled cDNA oligomer is a biotin-labeled cDNA prepared from a biotin-labeled primer. The microarray is then processed by direct detection of the biotin-containing transcripts using, e.g., Streptavidin-Alexa647 conjugate, and scanned utilizing conventional scanning methods. Image intensities of each spot on the array are proportional to the abundance of the corresponding biomarker in the subject sample.

The use of the array has one or more advantages for mRNA expression detection. First, the global expression of several to thousands of genes can be identified in a single sample at one time. Second, through careful design of the oligonucleotide probes, the expression of both mature and precursor molecules can be identified. Third, in comparison with Northern blot analysis, the chip requires a small amount of RNA.

The ASRGL1 mRNA may for example be extracted from formalin-fixed, paraffin-embedded tumor tissue. Accordingly, the sample of the methods of the present disclosure may be formalin-fixed and/or paraffin-embedded endometrial tumor tissue independent of if ASRGL1 protein or ASRGL1 mRNA is detected.

The inventors have realized that ASRGL1 mRNA analysis of the present disclosure may be incorporated in an mRNA-based assay designed to support individualized treatment planning. Such an assay may employ RT-PCR to analyze the expression of several genes.

To perform the methods of the present disclosure, a kit may be employed. There is thus provided a kit for selecting a treatment or establishing a prognosis for an endometrial cancer subject, which kit comprises
a) a quantifiable affinity ligand capable of selective interaction with a ASRGL protein;
b) reagents necessary for quantifying the amount of the quantifiable affinity ligand of a).

Various components of the kit may be selected and specified as described above in connection with the method aspects of the present disclosure.

Thus, the kit according to the present disclosure comprises an affinity ligand against ASRGL1 protein, as well as other means that help to quantify the specific and/or selective affinity ligand after they have bound specifically and/or selectively to the respective target proteins. For example, the kit may contain a secondary affinity ligand for detecting and/or quantifying a complex formed by the target protein and the affinity ligand. The kit may also contain various auxiliary substances other than the affinity ligand, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g., endogenous enzyme block solution to decrease the background staining and/or counterstaining solution to increase staining contrast, that are commonly used in immunoassay reagent kits.

In embodiments of the kit, the affinity ligand may be selected as described above in connection with the method aspects.

The kit may also advantageously comprise a reference sample for provision of, or yielding, the reference value to be used for comparison with the sample value. For example, the reference sample may comprise a predetermined amount of ASRGL1. Such a reference sample may for example be constituted by a tissue sample containing the predetermined amount of ASRGL1 protein. The tissue reference sample may then be used by the person of skill in the art in the determination of the ASRGL1 expression status in the sample being studied, by manual, such as ocular, or automated comparison of expression levels in the reference tissue sample and the subject sample. As another example, the reference sample may comprise cell lines, such as cancer cell lines, expressing a predetermined, or controlled, amount of ASRGL1. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

One or more pictures may also be provided as the "reference sample". For example, the picture may show an example of a tumor tissue slide stained with a certain antibody during certain conditions and exhibiting a certain cytoplasmic intensity and/or fraction. The above discussion about the "reference sample" applies *mutatis mutandis* to pictures.

As a third aspect of the present disclosure, there is provided a use of an ASRGL1 protein or an ASRGL1 mRNA molecule for identifying a favorable endometrial cancer prognosis. The use may be *ex vivo.* The ASRGL1 protein or ASRGL1 mRNA molecule of the third aspect may for example be provided in a tumor tissue sample from the endometrial glands.

As a fourth aspect of the present disclosure, there is provided an affinity ligand capable of selective interaction with an ASRGL1 protein for identifying a favorable endometrial cancer prognosis. Different embodiments of such an affinity ligand are discussed above in connection with the method aspects.

The favorable endometrial cancer prognosis of the third or fourth aspect may for example be an expected five-year disease-free survival of at least 80 %, such as at least 85 %, such as at least 90 %. A subject having such a favorable prognosis may be relieved from any adjuvant treatment, in particular if the tumor is stage I and grade 1 or 2.

In the context of the present disclosure, "ASRGL1" refers to ASRGL1 protein or ASRGL1 mRNA. However, the inventors generally believe that the ASRGL1 protein expression is more relevant than the ASRGL1 mRNA expression. Thus, "ASRGL1" preferably refers to ASRGL1 protein.

### Example

### Endometrial cancer TMA

### a) Material and methods

Tumor material was collected from 220 patients diagnosed with endometrial cancer of the endometroid type at the Turku University Hospital between 2004 and 2007. The median age of patients was 67 (35-93) years. There were 118 patients with grade 1 tumors, 62 patients with grade 2 tumors, and 38 patients with grade 3 tumors. Myometrial invasion above 50 % was present in tumors of 66 patients.

Prior to TMA construction, all available haematoxylin and eosin stained slides from each case were histopathologically re-evaluated. A standard set of 4x1 mm cores were taken from each invasive tumor in a proportional fashion, covering up to 3 different components. A semi-automated arraying device was used (TMArrayer; Pathology Devices, Inc, Westminster, MD, USA).

All immunohistochemical stainings were performed in the PT-link system (DAKO, Copenhagen, Denmark), including automated pre-treatment. The slides were incubated for 30 min at room temperature with the primary anti-ASRGL1 polyclonal antibody HPA029725 (Atlas Antibodies, Stockholm, Sweden). The slides were further incubated with the secondary reagent, anti-rabbit/mouse horse reddish peroxidase-conjugated UltraVision (Thermo Fisher Scientific, Runcorn, UK) for 30 min at room temperature. Between all steps, slides were rinsed in wash buffer (Dako). Finally, diaminobenzidine (Dako) was used as chromogen and Harris hematoxylin (Sigma-Aldrich) was used for counterstaining. The slides were mounted with Pertex® (Histolab).

All samples of immunohistochemically stained tissue were manually evaluated under the microscope and annotated by a certified pathologist. Annotation of each sample was performed using a simplified scheme for classification of IHC outcome. Each tissue sample was examined for representativity and immunoreactivity.

Basic annotation parameters included an evaluation of i) subcellular localization (nuclear and/or cytoplasmic/membranous), ii) cytoplasmaic staining intensity (CI) and iii) fraction of cells with cytoplasmic staining (CF). Staining intensity was subjectively evaluated in accordance to standards used in clinical histo-pathological diagnostics and outcome was classified as: negative = no immunoreactivity, weak = faint immunoreactivity, moderate = medium immunoreactivity, or strong = distinct and strong immunoreactivity. Also fraction of stained cells was subjectively evaluated in accordance to standards used in clinical histo-pathological diagnostics and outcome was classified as: < 2 %, 2 - 10 %, 11 - 25 %, 26 - 50 %, 51 - 75 %, or > 75 % immunoreactive cells of the relevant cell population. The skilled artisan will recognize that this annotation procedure is similar to a calculation of an Allred score, see e.g. Allred et al (1998) Mod Pathol 11 (2), 155.

For graphic presentation and statistical analysis, dichotomized variables were used, where CI and/or CF were grouped to yield for example negative vs positive CI, or CF < 2 % vs CF ≥ 2 %. CI and CF were also combined by multiplying CI and CF scores, to yield a staining score (SS) ranging from 1 to 24.

The above classifications of samples were used for disease free survival (DFS) analysis according to the Kaplan-Meier method, and the log-rank test was used to compare survival in different strata. All statistical tests were two-sided, and p-values of < 0.05 % were considered significant. All calculations were made with the statistical package SPSS 20.0 (SPSS Inc. Illinois, USA).

### b) Results

The level of ASRGL1 expression was correlated to the disease free survival of endometrial cancer patients as can be seen in Figure 1. Similar results were obtained with both CI (Figure 1A) and CF (Figure 1 B) as a measure of ASRGL1 protein level. As can be seen in Figure 2, a negative-positive cut-off value for both CI (Figure 2A) and CF (Figure 2B) resulted in two patient groups of significantly different disease free survival prognoses. The prognostic value of ASRGL1 was found to be independent of stage and grade of the tumor. Figure 3 (A-C) shows the grade independent prognostic value of ASRGL1 protein with a CF cut-off of 75 % positive cells. Figure 4 (A-C) shows the grade independent prognostic value of ASRGL1 protein with a CI cut-off of negative vs. positive tumors. In Figure 5 (A-D), the disease free survival of patients in the cohort can be seen for different stages of endometrial cancer. For stage I and II (Figure 5A and B, respectively), there was a significantly lower survival for patients with lower levels of the ASRGL1 protein. For stage III (Figure 5C), the difference in survival did not reach a significant level, but still a clear trend can be seen. For stage IV (Figure 5D), no conclusions could be drawn due to no cases with CF > 75 % in this group.

When considering the perhaps most interesting group of patients, those considered to belong to the intermediate risk group, Figure 6 shows disease free survival for intermediate risk patients with tumors of all stages, corresponding to pre-operative risk assessment. Figure 6A shows the impact of ASRGL1 protein level on patient survival, with protein level measured by CI with a cut-off of negative vs. positive. Figure 6B shows the impact of ASRGL1 protein level on patient survival with protein level measured by CF with a cut-off of 1 % positive cells. When considering only those intermediate risk patients with grade 1 or grade 2 tumors and a myometrial invasion of > 50%, there is an even more profound difference in survival between patients whose tumors express low levels of ASRGL1 protein and patients whose tumors express high levels of ASRGL1 protein (Figure 7), suggesting that ASRGL1 level might be particularly interesting in this subgroup. Figure 8 shows disease free survival for intermediate risk patients with stage I tumors, corresponding to post-operative risk assessment. Figure 8A shows the impact of ASRGL1 protein level on survival for all patients with stage I endometrial cancer in the cohort as measured by CF with at cut-off of 75 % positive tumor cells. Figure 8B shows the impact of ASRGL1 protein level on patient survival for stage I patients with grade 1 or grade 2 tumors and a myometrial invasion of > 50%.

It is also possible to combine CI and CF into a single variable by e.g. mulitiplying the score of CI and CF yielding a staining score (SS) from 1 to 24, where a SS = 1 corresponds to no ASRGL1 protein expression. Figure 9 shows the impact of ASRGL1 protein level on patient survival for patients with grade 1 or grade 2 tumors and a myometrial invasion of > 50% using SS with a cut-off of negative (SS=1) vs. positive (SS>1).

### A non-limiting example of an establishment of a prognosis

Following abnormal uterine bleeding in a postmenopausal woman, transvaginal ultrasound is performed in order to determine the endometrium thickness. If the endometrium is thicker than 4 mm, or immeasurable, it is concluded that a tumor may be present, and an endometrial biopsy is normally performed. The biopsy material is analyzed morphologically, and if endometrial cancer is confirmed, further diagnostic imaging is performed to assess myometrial infiltration and potential presence of metastatic spread. In case of cancer, the carcinomas is graded according to the FIGO system and preoperatively stratified into risk groups.

Further, tumor tissue sample from the biopsy is used for determining the level of ASRGL1 protein according to the present disclosure. For the provision of a "negative reference", a sample is taken from archival material comprising tissue essentially lacking ASRGL1 protein expression. Such archival tissue may for example be tissue having a pre-established absent ASRGL1 protein expression. Further, for the provision of a "positive reference", a sample is taken from archival material comprising tissue having high ASRGL1 protein expression, such as tissue having a pre-established high (e.g. NF > 75 %) ASRGL1 protein expression level.

The sample material is fixated in buffered formalin and histo-processed in order to obtain thin sections (e.g. 4 µm) of the sample material.

One or more sample sections from each sample is/are mounted on glass slides that are incubated for 45 min in 60 °C, de-paraffinized (if the sample in question was paraffinized) in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections is to increase the accuracy of the results.

The slides are incubated for 30 min at room temperature with a primary anti-ASRGL1 polyclonal antibody HPA029725 (Atlas Antibodies, Stockholm, Sweden). The slides are further incubated with the secondary reagent, anti-rabbit/mouse horse reddish peroxidase-conjugated UltraVision (Thermo Fisher Scientific, Runcorn, UK) for 30 min at room temperature. Between all steps, slides are rinsed in wash buffer (Dako). Finally, diaminobenzidine (Dako) is used as chromogen and Harris hematoxylin (Sigma-Aldrich) is used for counterstaining. The slides were mounted with Pertex® (Histolab) mounting media.

As a tool to validate the staining procedure, two control cell-lines may be used; e.g. one slide with cells expressing ASRGL1 protein (positive cell line) and one slide having cells with no ASRGL1 protein expression (negative cell line). The skilled artisan understands how to provide such cell lines, for example guided by the disclosure of Rhodes et al. (2006) The biomedical scientist, p 515-520. The control-line slides may be simultaneously stained in the same procedure as the other slides, i.e. incubated with the same primary and secondary antibodies.

For example, the tumor tissue slides from the subject, the staining reference slides, and optionally, the slides with control cell-lines, may be scanned in a light microscope using a ScanScope T2 automated slide scanning system (Aperio Technologies) at x20 magnification. However, this scanning step is not necessary, but may make the procedure easier if, for example, the preparation and staining of the slides and the evaluation of the stained slides (see below) are performed at different locations or by different persons.

If control cell-lines are used, these are inspected to validate the staining procedure. If the cell-lines display staining results outside acceptable criteria, e.g. staining artifacts recognized by the skilled artisan, the staining of the tissue samples is considered invalid and the whole staining procedure is repeated with new slides. If the positive and negative cell-lines display strong staining intensity and no staining intensity, respectively, the staining is considered as valid.

The stained sample slide(s) from the tumor tissue sample from the patient is/are manually evaluated by visual inspection, and sample values (cytoplasmic intensity (CI) and cytoplasmic fraction (CF)) of each slide are determined according to the above. The person performing the evaluation and determination is aided by visual inspection of the stained positive and negative reference slides.

The sample value(s) from the tumor tissue sample from the patient is/are then compared to a reference value. If more than one sample slide are evaluated and thereby more than one sample value are obtained, the sample value that is compared to the reference value may be a mean or median value of the obtained sample values.

The reference value may be an absent/negative CI. In such case it is concluded that the tested patient belongs to a group of patients having a relatively good prognosis if the CI of the sample(s) is positive (i.e. weak, moderate or strong) and a group of patients having a relatively poor prognosis if the CI of the sample(s) is absent/negative. The prognoses of the respective groups, may be read from dichotomized data as those presented in the figures, wherein the upper curve represents the group of patients having the relatively good prognosis and the lower curve represents the group of patients having the relatively poor prognosis. For example, the relatively good prognosis may be an average five-year disease free survival of about 93 % and the relatively poor prognosis may be an average five-year disease free survival of about 50 % (figure 2A).

If the above-mentioned grading and myometrial infiltration assessment revealed that the tumor is grade 1 or 2 and the myometrial invasion is > 50%, the result of the prognostic method may be used to determine the extent of surgery (see fig 7A), wherein a negative CI may favor lymphadenectomy, while a positive CI supports refraining from lymphadenectomy.

After surgery, removed tissue material is used for grading and staging according to the FIGO-system as well as myometrial infiltration assessment and a new ASRGL1 measurement performed as described above. Thus, the stratification into risk groups is reevaluated. The risk group in combination with the prognostic information from the ASRGL1 measurement is used for determining an appropriate adjuvant treatment regimen. For example, if the reevaluation reveals that the tumor is stage I and grade 1 or 2 and that the myometrial invasion is > 50% (fig 8B), it may be concluded that the subject shall be treated by vaginal brachytherapy if the sample value(s) is/are higher than the reference value CF = 75 % and to refrain from any adjuvant treatment if the sample value(s) is/are lower than or equal to CF = 75 %.

All cited material, including but not limited to publications, DNA or protein data entries, and patents, referred to in this application are herein incorporated by reference.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. Method for determining whether a mammalian subject having an endometrial cancer belongs to a first or a second group, wherein the prognosis of subjects of the first group is better than the prognosis of subjects of the second group, comprising the steps of:
a) evaluating an amount of ASRGL1 in at least part of a sample earlier obtained from the subject and determining a sample value corresponding to the evaluated amount;
b) comparing said sample value with a predetermined reference value; and
if said sample value is higher than said reference value,
c1) concluding that the subject belongs to the first group; and
if said sample value is lower than or equal to said reference value,
c2) concluding that the subject belongs to the second group.

2. Method according to claim 1, wherein the prognosis is a grade-independent and/or stage-independent prognosis.

3. Method for determining whether a subject having an endometrial cancer is not in need of an endometrial cancer treatment regimen, comprising the steps of:
a) evaluating an amount of ASRGL1 in at least part of a sample earlier obtained from the subject and determining a sample value corresponding to the evaluated amount;
b) comparing said sample value with a predetermined reference value; and
if said sample value is higher than said reference value,
c) concluding that said subject is not in need of the endometrial cancer treatment regimen.

4. Non-treatment strategy method for a subject having an endometrial cancer, comprising the steps of:
a) evaluating an amount of ASRGL1 in at least part of a sample earlier obtained from the subject and determining a sample value corresponding to the evaluated amount;
b) comparing said sample value with a predetermined reference value; and
if said sample value is higher than said reference value,
c) refraining from treating said subject with an endometrial cancer treatment regimen.

5. Method according to claim 4, wherein the endometrial cancer treatment regimen comprises adjuvant chemotherapy.

6. Method according to claim 4 or 5, wherein the endometrial cancer treatment regimen comprises adjuvant radiation therapy, such as vaginal brachytherapy.

7. Method for determining whether a first or a second number of lymph nodes shall be removed from a mammalian subject having an endometrial cancer, wherein the first number is higher than the second number, comprising the steps of:
a) evaluating an amount of ASRGL1 in at least part of a sample earlier obtained from the subject and determining a sample value corresponding to the evaluated amount;
b) comparing said sample value with a predetermined reference value; and
if said sample value is higher than said reference value,
c1) concluding that the second number of lymph nodes shall be removed; and
if said sample value is lower than or equal to said reference value,
c2) concluding that the first number of lymph nodes shall be removed.

8. Method according to any one of the preceding claims, wherein the endometrial cancer is grade 1 or 2 with more than 50 % myometrial infiltration or grade 3 with less than 50 % myometrial infiltration.

9. Method according to any one of the preceding claims, wherein the endometrial cancer is stage I.

10. Method according to any one of the preceding claims, wherein the sample comprises endometrial cancer tumor cells from said subject.

11. Method according to any one of the preceding claims, wherein the sample is an endometrial tumor tissue sample.

12. Use *ex vivo* of an ASRGL1 protein or an ASRGL1 mRNA molecule for identifying a favorable endometrial cancer prognosis.

13. Use *ex vivo* of an affinity ligand capable of selective interaction with an ASRGL1 protein for identifying a favorable endometrial cancer prognosis.

14. Use according to claim 13, wherein the affinity ligand is selected from the group consisting of antibodies and Fab fragments, Fv fragments and single chain Fv (scFv) fragments thereof.

15. Use according to any one of claims 12-14, wherein the favorable endometrial cancer prognosis is an expected five-year survival of at least 80 %, such as at least 85 %, such as at least 90 %.


SEQUENCE LISTING

<110>  Atlas Atibodies AB

<120>  ASRGL1 IN ENDOMETRIAL CANCER

<130>  13074EP

<160>  5

<170>  PatentIn version 3.5

<210>  1
<211>  82
<212>  PRT
<213>  Homo sapiens

<400>  1

Met Asp Gly Lys Asp Leu Ser Ala Gly Ala Val Ser Ala Val Gln Cys
1               5                   10                  15

Ile Ala Asn Pro Ile Lys Leu Ala Arg Leu Val Met Glu Lys Thr Pro
            20                  25                  30

His Cys Phe Leu Thr Asp Gln Gly Ala Ala Gln Phe Ala Ala Ala Met
        35                  40                  45

Gly Val Pro Glu Ile Pro Gly Glu Lys Leu Val Thr Glu Arg Asn Lys
    50                  55                  60

Lys Arg Leu Glu Lys Glu Lys His Glu Lys Gly Ala Gln Lys Thr Asp
65                  70                  75                  80

Cys Gln

<210>  2
<211>  308
<212>  PRT
<213>  Homo sapiens

<400>  2

Met Asn Pro Ile Val Val Val His Gly Gly Gly Ala Gly Pro Ile Ser
1               5                   10                  15

Lys Asp Arg Lys Glu Arg Val His Gln Gly Met Val Arg Ala Ala Thr
            20                  25                  30

Val Gly Tyr Gly Ile Leu Arg Glu Gly Gly Ser Ala Val Asp Ala Val
        35                  40                  45

Glu Gly Ala Val Val Ala Leu Glu Asp Asp Pro Glu Phe Asn Ala Gly

50                  55                  60

Cys Gly Ser Val Leu Asn Thr Asn Gly Glu Val Glu Met Asp Ala Ser
65                  70                  75                  80

Ile Met Asp Gly Lys Asp Leu Ser Ala Gly Ala Val Ser Ala Val Gln
                85                  90                  95

Cys Ile Ala Asn Pro Ile Lys Leu Ala Arg Leu Val Met Glu Lys Thr
            100                 105                 110

Pro His Cys Phe Leu Thr Asp Gln Gly Ala Ala Gln Phe Ala Ala Ala
        115                 120                 125

Met Gly Val Pro Glu Ile Pro Gly Glu Lys Leu Val Thr Glu Arg Asn
    130                 135                 140

Lys Lys Arg Leu Glu Lys Glu Lys His Glu Lys Gly Ala Gln Lys Thr
145                 150                 155                 160

Asp Cys Gln Lys Asn Leu Gly Thr Val Gly Ala Val Ala Leu Asp Cys
                165                 170                 175

Lys Gly Asn Val Ala Tyr Ala Thr Ser Thr Gly Gly Ile Val Asn Lys
            180                 185                 190

Met Val Gly Arg Val Gly Asp Ser Pro Cys Leu Gly Ala Gly Gly Tyr
        195                 200                 205

Ala Asp Asn Asp Ile Gly Ala Val Ser Thr Thr Gly His Gly Glu Ser
    210                 215                 220

Ile Leu Lys Val Asn Leu Ala Arg Leu Thr Leu Phe His Ile Glu Gln
225                 230                 235                 240

Gly Lys Thr Val Glu Glu Ala Ala Asp Leu Ser Leu Gly Tyr Met Lys
                245                 250                 255

Ser Arg Val Lys Gly Leu Gly Gly Leu Ile Val Val Ser Lys Thr Gly
            260                 265                 270

Asp Trp Val Ala Lys Trp Thr Ser Thr Ser Met Pro Trp Ala Ala Ala
        275                 280                 285

Lys Asp Gly Lys Leu His Phe Gly Ile Asp Pro Asp Asp Thr Thr Ile
    290                 295                 300

Thr Asp Leu Pro
305

<210> 3
<211> 180
<212> PRT
<213> Homo sapiens

<400> 3

Met Gly Val Pro Glu Ile Pro Gly Glu Lys Leu Val Thr Glu Arg Asn
1               5                   10                  15

Lys Lys Arg Leu Glu Lys Glu Lys His Glu Lys Gly Ala Gln Lys Thr
            20                  25                  30

Asp Cys Gln Lys Asn Leu Gly Thr Val Gly Ala Val Ala Leu Asp Cys
        35                  40                  45

Lys Gly Asn Val Ala Tyr Ala Thr Ser Thr Gly Gly Ile Val Asn Lys
    50                  55                  60

Met Val Gly Arg Val Gly Asp Ser Pro Cys Leu Gly Ala Gly Gly Tyr
65                  70                  75                  80

Ala Asp Asn Asp Ile Gly Ala Val Ser Thr Thr Gly His Gly Glu Ser
                85                  90                  95

Ile Leu Lys Val Asn Leu Ala Arg Leu Thr Leu Phe His Ile Glu Gln
            100                 105                 110

Gly Lys Thr Val Glu Glu Ala Ala Asp Leu Ser Leu Gly Tyr Met Lys
        115                 120                 125

Ser Arg Val Lys Gly Leu Gly Gly Leu Ile Val Val Ser Lys Thr Gly
    130                 135                 140

Asp Trp Val Ala Lys Trp Thr Ser Thr Ser Met Pro Trp Ala Ala Ala
145                 150                 155                 160

Lys Asp Gly Lys Leu His Phe Gly Ile Asp Pro Asp Asp Thr Thr Ile
                165                 170                 175

Thr Asp Leu Pro
            180

<210> 4
<211> 2424
<212> DNA
<213> Homo sapiens

<400> 4
gttccccgcg tgccaccagg aagctcgggc cggccaagag cgtagactct tgagaggagt 60
gagacaggtg cgcgccagcc ggccttcggg gctttatggg aactgggccg tgcggcggtc 120
ccgccctcgt gcgcaggcgc agaaccgttg tgaccagagc ggttgcgggc tgagcggttt 180
cgagccggcg tcggggagcg gcggtaccgg gcggctgcgg ggctggctcg acccagcttg 240
aggtctcggc gtccgcgtcc tgcggtgccc tggggtctcc cgaggacctt gtacccgcgc 300
ggcttccttg ggctggcttt ggacgacgct ttcgccttcc tgctgcctag gatccgccga 360
catgaatccc atcgtagtgg tccacggcgg cggagccggt cccatctcca aggatcggaa 420
ggagcgagtg caccagggca tggtcagagc cgccaccgtg ggctacggca tcctccggga 480
gggcgggagc gccgtggatg ccgtagaggg agctgtcgtc gccctggaag acgatcccga 540
gttcaacgca ggttgtgggt ctgtcttgaa cacaaatggt gaggttgaaa tggatgctag 600
tatcatggat ggaaaagacc tgtctgcagg agcagtgtcc gcagtccagt gtatagcaaa 660
tcccattaaa cttgctcggc ttgtcatgga aaagacacct cattgctttc tgactgacca 720
aggcgcagcg cagtttgcag cagctatggg ggttccagag attcctggag aaaaactggt 780
gacagagaga aacaaaaagc gcctggaaaa agagaagcat gaaaaaggtg ctcagaaaac 840
agattgtcaa aaaaacttgg gaaccgtggg tgctgttgcc ttggactgca aagggaatgt 900
agcctacgca acctccacag gcggtatcgt taataaaatg gtcggccgcg ttggggactc 960
accgtgtcta ggagctggag gttatgccga caatgacatc ggagccgtct caaccacagg 1020
gcatggggaa agcatcctga aggtgaacct ggctagactc accctgttcc acatagaaca 1080
aggaaagacg gtagaagagg ctgcggacct atcgttgggt tatatgaagt caagggttaa 1140
aggtttaggt ggcctcatcg tggttagcaa aacaggagac tgggtggcaa agtggacctc 1200
cacctccatg ccctgggcag ccgccaagga cggcaagctg cacttcggaa ttgatcctga 1260
cgatactact atcaccgacc ttccctaagc cgctggaaga ttgtattcca gatgctagct 1320
tagaggtcaa gtacagtctc ctcatgagac atagcctaat caattagatc tagaattgga 1380
aaaattgtcc cgtctgtcac ttgttttgtt gccttaataa gcatctgaat gtttggttgt 1440
ggggcgggtt ctgaagcgat gagagaaatg cccgtattag gaggattact tgagcccagg 1500
aggtcaaagc tgaggtgagc catgattact ccactgcact ccagcctggg caacagagcc 1560
aggccctgta tcaaaaaaaa aaaaaaaaag aaaagggaaa aaagaaagaa agcagcagca 1620
tgatcctgac atgacagatg tgggagaccc acagcctgca gacactgtgg gctggaaggt 1680
gggaagggag gggccggtgg aggtggagct gtttgaaagt gacacagcag cagtagaagc 1740
agtggtgggc gaagcccagg tgaccctcag aacgttgcac aagaacatca gggaaaagaa 1800
ccagaatcct ttaaggaaaa tgttcttcat gtatgagaga ctaaagtgat ttttctaaga 1860

aagttcagcc cttctctgac ttacctggac atttctagat acttccaaag gaccctctgg 1920

gaatccatag cttcctaatc tggagatggg aggtcataag ggagacgctg tggggttcct 1980

tgaagtttct tgggttcaca gaggagcccc ctcacttggt gttctcccgt gagccagcct 2040

ccacctgcca aagacactct ggtcctcgta tagtgagtaa tggggctcag ggcctctcca 2100

acaacagaga ggagctgatg ctgtagggct gaccccgtga cttcctgagt cctcaccctg 2160

tccagtgctt tgagattctt cccacctccc catcctcacc agccggatcg ggcgctgtgc 2220

agtgtggtca gcatggtgaa gaaagtcatt tcctcggtgg gcagtattcc tctttatctc 2280

tcattacact ggaaatgtta tttctgctgt atcatccgtg ctcaacgttt tagtctgtca 2340

ggctcacctt ctctctggaa agaatttgct taacttgaca ttccatgtgc cgctaataaa 2400

atatattttg aaagaataaa aaaa 2424

<210> 5
<211> 2347
<212> DNA
<213> Homo sapiens

<400> 5
gttccccgcg tgccaccagg aagctcgggc cggccaagag cgtagactct tgagaggagt 60

gagacaggtg cgcgccagcc ggccttcggg gctttatggg aactgggccg tgcggcggtc 120

ccgccctcgt gcgcaggcgc agaaccgttg tgaccagagc ggttgcgggc tgagcggttt 180

cgagccggcg tcggggagcg gcggtaccgg gcggctgcgg ggctggctcg acccagcttg 240

aggtctcggc gtccgcgtcc tgcggtgccc tgggatccgc cgacatgaat cccatcgtag 300

tggtccacgg cggcggagcc ggtcccatct ccaaggatcg gaaggagcga gtgcaccagg 360

gcatggtcag agccgccacc gtgggctacg gcatcctccg ggagggcggg agcgccgtgg 420

atgccgtaga gggagctgtc gtcgccctgg aagacgatcc cgagttcaac gcaggttgtg 480

ggtctgtctt gaacacaaat ggtgaggttg aaatggatgc tagtatcatg gatggaaaag 540

acctgtctgc aggagcagtg tccgcagtcc agtgtatagc aaatcccatt aaacttgctc 600

ggcttgtcat ggaaaagaca cctcattgct ttctgactga ccaaggcgca gcgcagtttg 660

cagcagctat gggggttcca gagattcctg gagaaaaact ggtgacagag agaaacaaaa 720

agcgcctgga aaaagagaag catgaaaaag gtgctcagaa aacagattgt caaaaaaact 780

tgggaaccgt gggtgctgtt gccttggact gcaaagggaa tgtagcctac gcaacctcca 840

caggcggtat cgttaataaa atggtcggcc gcgttgggga ctcaccgtgt ctaggagctg 900

gaggttatgc cgacaatgac atcggagccg tctcaaccac agggcatggg gaaagcatcc 960

tgaaggtgaa cctggctaga ctcaccctgt tccacataga acaaggaaag acggtagaag 1020

aggctgcgga cctatcgttg ggttatatga agtcaagggt taaaggttta ggtggcctca 1080

tcgtggttag caaaacagga gactgggtgg caaagtggac ctccacctcc atgccctggg 1140

cagccgccaa ggacggcaag ctgcacttcg gaattgatcc tgacgatact actatcaccg 1200

accttcccta agccgctgga agattgtatt ccagatgcta gcttagaggt caagtacagt 1260

ctcctcatga gacatagcct aatcaattag atctagaatt ggaaaaattg tcccgtctgt 1320

cacttgtttt gttgccttaa taagcatctg aatgtttggt tgtggggcgg gttctgaagc 1380

gatgagagaa atgcccgtat taggaggatt acttgagccc aggaggtcaa agctgaggtg 1440

agccatgatt actccactgc actccagcct gggcaacaga gccaggccct gtatcaaaaa 1500

aaaaaaaaaa aagaaaaggg aaaaaagaaa gaaagcagca gcatgatcct gacatgacag 1560

atgtgggaga cccacagcct gcagacactg tgggctggaa ggtgggaagg gaggggccgg 1620

tggaggtgga gctgtttgaa agtgacacag cagcagtaga agcagtggtg ggcgaagccc 1680

aggtgaccct cagaacgttg cacaagaaca tcagggaaaa gaaccagaat cctttaagga 1740

aaatgttctt catgtatgag agactaaagt gatttttcta agaaagttca gcccttctct 1800

gacttacctg gacatttcta gatacttcca aaggaccctc tgggaatcca tagcttccta 1860

atctggagat gggaggtcat aagggagacg ctgtggggtt ccttgaagtt tcttgggttc 1920

acagaggagc cccctcactt ggtgttctcc cgtgagccag cctccacctg ccaaagacac 1980

tctggtcctc gtatagtgag taatggggct cagggcctct ccaacaacag agaggagctg 2040

atgctgtagg gctgaccccg tgacttcctg agtcctcacc ctgtccagtg ctttgagatt 2100

cttcccacct ccccatcctc accagccgga tcgggcgctg tgcagtgtgg tcagcatggt 2160

gaagaaagtc atttcctcgg tgggcagtat tcctctttat ctctcattac actggaaatg 2220

ttatttctgc tgtatcatcc gtgctcaacg ttttagtctg tcaggctcac cttctctctg 2280

gaaagaattt gcttaacttg acattccatg tgccgctaat aaaatatatt ttgaaagaat 2340

aaaaaaa 2347